# EUROPEAN PATENT SPECIFICATION

(11) **EP 0 680 316 B1**
(45) Date of publication and mention of the grant of the patent: **10.12.2003**
(21) Application number: 94907845.5
(22) Date of filing: 12.01.1994
(51) Int. Cl.: C12N 15/12, C07K 14/435, C07K 16/18, A61K 38/17

(54) **NOVEL PARASITIC HELMINTH PROTEINS**
NEUE PROTEINE VON PARASITISCHEN WÜRMERN
NOUVELLES PROTEINES PARASITAIRES HELMINTHIQUES

(30) Priority: 12.01.1993 US 3257; 12.01.1993 US 3389; 19.08.1993 US 109391
(43) Date of publication of application: 08.11.1995
(73) Proprietor: Heska Corporation, Fort Collins, CO 80526 (US); Colorado State University Research Foundation, Fort Collins, CO 80521 (US)
(72) Inventor: GRIEVE, Robert B., Windsor, CO 80550 (US); FRANK, Glenn R., Fort Collins, CO 80524 (US); MIKA-GRIEVE, Marcia, Windsor, CO 80550 (US); TRIPP, Cynthia Ann, Fort Collins, CO 80525 (US)
(74) Representative: Harding, Charles Thomas
(86) International application number: PCT/US94/00679
(87) International publication number: WO 94/015593

(56) References cited:
- WO-A-92/13560
- C.B. POOLE ET AL.: "Cloning of a cuticular antigen that contains multiple tandem repeats from the filarial parasite Dirofilaria immitis" PROC. NATL. ACAD. SCI. USA, vol. 89, July 1992, pages 5986-5990, XP000647858
- S. SUN ET AL.: "Stage-specific expression of a deveopmentally regulated gene in Dirofilaria immitis" JOURNAL OF HELMINTHOLOGY, vol. 66, no. 1, 1992, pages 62-67, XP000646848
- R.J. LIMBERGER ET AL.: "Filarial paramyosin: cDNA sequences from Dirofilaria immitis and Onchocerca volvulus" MOLECULAR AND BIOCHEMICAL PARASITOLOGY, vol. 38, no. 2, 15 January 1990, pages 271-280, XP000646806
- LAL R B ET AL: "CHARACTERIZATION OF STAGE-SPECIFIC ANTIGENS OF INFECTIVE LARVAE OF THE FILARIAL PARASITE BRUGIA-MALAYI." J IMMUNOL 140 (6). 1988. 2032-2038. CODEN: JOIMA3 ISSN: 0022-1767, XP002044036
- M. PHILIPP ET AL.: "Biochemical and immunolgic characterization of a major surface antigen of Dirofilaria immitis infective larvae." JOURNAL OF IMMUNOLOGY, vol. 136, no. 7, 1 April 1986, pages 2621-2627, XP002044037
- A.L. SCOTT ET AL.: "Surface-associated antigens of the second, third and fourth larvae of Dirofilaria immitis" ACTA TROPICA, vol. 47, 1990, pages 339-353, XP002044038
- M.S. IBRAHIM ET AL.: "Antigen shedding from the surface of the infective stage larvae of Dirofilaria immitis" PARASITOLOGY, vol. 99, 1989, pages 89-97, XP002044039
- Molecular and Biochemical Parasitology, Volume 54, issued 1992, CULPEPPER et al., "Molecular Characterization of a Dirofilaria Immitis cDNA Encoding a Highly Immunoreactive Antigen", pages 51-62, see Abstract, pages 53-55.

## Description

### Field of the Invention

The present invention relates to novel parasitic helminth proteins, nucleic acid sequences encoding such proteins and antibodies raised against such proteins. The present invention also includes a method to obtain such nucleic acid sequences and proteins, and a method of using such nucleic acid sequences, antibodies, and proteins to protect animals from infection. The present invention particularly relates to specific *Dirofilaria immitis* (*D. immitis*) nucleic acid sequences and proteins as well as their use to protect animals from heartworm infection.

### Background of the Invention

Parasitic helminth infections in animals, including humans, are typically treated by chemical drugs, because there are essentially no efficacious vaccines available. One disadvantage with chemical drugs is that they must be administered often. For example, dogs susceptible to heartworm are typically treated monthly to maintain protective drug levels. Repeated administration of drugs to treat parasitic helminth infections, however, often leads to the development of resistant strains that no longer respond to treatment. Furthermore, many of the chemical drugs are harmful to the animals being treated, and as larger doses become required due to the build up of resistance, the side effects become even greater.

It is particularly difficult to develop vaccines against parasitic helminth infections both because of the against parasitic helminth infections both because of the complexity of the parasite's life cycle and because, while administration of parasites or parasite antigens can lead to the production of a significant antibody response, the immune response is typically not sufficient to protect the animal against infection.

As for most parasites, the life cycle of *D. immitis*, the helminth that causes heartworm, includes a variety of life forms, each of which presents different targets, and challenges, for immunization. Adult forms of the parasite are quite large and preferentially inhabit the heart and pulmonary arteries of an animal. Males worms are typically about 12 cm (centimeters) to about 20 cm long and about 0.7 mm to about 0.9 mm wide; female worms are about 25 cm to about 31 cm long and about 1.0 to about 1.3 mm wide. Sexually mature adults, after mating, produce microfilariae which are only about 300 µm (micrometers) long and about 7 µm wide. The microfilariae traverse capillary beds and circulate in the vascular system of the dog in concentrations of about 10³ to about 10⁵ microfilariae per ml of blood. One method of demonstrating infection in the dog is to detect the circulating microfilariae.

If the dog is maintained in an insect-free environment, the life cycle of the parasite cannot progress. However, when microfilariae are ingested by the female mosquito during blood feeding on an infected dog, subsequent development of the microfilariae into larvae occurs in the mosquito. The microfilariae go through two larval stages (L1 and L2) and finally become mature third stage larvae (L3) of about 1.1 mm length, which can then be transmitted back to the dog through the bite of the mosquito. It is this L3 stage, therefore, that accounts for the initial infection. As early as three days after infection, the L3 molt to the fourth larval (L4) stage, and subsequently to the fifth stage, or immature adults. The immature adults migrate to the heart and pulmonary arteries, where they mature and reproduce, thus producing the microfilariae in the blood. "Occult" infection with heartworm in dogs is defined as that wherein no microfilariae can be detected, but the existence of the adult heartworms can be determined through thoracic examination.

Heartworm not only is a major problem in dogs, which typically cannot even develop immunity upon infection (i.e., dogs can become reinfected even after being cured by chemotherapy), but is also becoming increasingly widespread in other companion animals, such as cats and ferrets. Heartworm infections have also been reported in humans. Other parasitic helminthic infections are also widespread, and all require better treatment, including a preventative vaccine program.

Although many investigators have tried to develop vaccines based on specific antigens, it is well understood that the ability of an antigen to stimulate antibody production does not necessarily correlate with the ability of the antigen to stimulate an immune response capable of protecting an animal from infection, particularly in the case of parasitic helminths. A large number of materials are immunogenic and produce sera which test positive in immunoassays for ability to react with the immunizing antigen, but which fail to protect the hosts against infection. Antibodies which neutralize the infective agent in *in vitro* assays are much more likely to protect against challenge *in vivo.* Accordingly, the use of serum simply resulting from immunization or from infection by a parasitic helminth to screen for candidate vaccines does not provide sufficient specificity to identify protective immunogens. On the other hand, serum or other components of blood from immunized animals which is demonstrably protective against infection would contain antibodies, cells, or other factors that could selectively bind to potential antigens that, if used as therapeutic compositions, would elicit immune responses that protect against challenge.

In most infectious diseases, particularly those such as parasitic infections that have long and complex development courses, it is difficult to verify the protective effect of serum or T-cells from exposed animals for use as a screening reagent. First, verification of protection against challenge is tedious, since the host animal would first have to be challenged with the infectious agent and shown to be protected before it could be shown that antibody components of serum, for example, could be used as a screen. The definition of protection under such a regimen is often complex. Second, even if a protective effect against challenge is shown, it is not clear to what components of the immune system the protection is due. The protective effect could be due to antibodies, cells, mediators of the immune system or to combinations thereof. Thus, although this method of obtaining the screening reagent is sometimes used, it is time-consuming and does not permit identification of protective components.

A method to determine the effectiveness of *in vivo* immunization protocols includes implanting diffusion chambers containing infectious agents into immunized animals and determining the effects of such immunizations on the implanted infectious agents. Grieve, et al., 1988, *Am. J. Trop. Med. Hyg.* 39, p. 373-379, for example, report that dogs which had been immunized against *D. immitis* infection were supplied diffusion chambers containing infective larvae. The larvae in the chambers could then be evaluated for the effect of the previous immunizations. Abraham, et al., 1988 *J. Parasit.* 74, p. 275-282, report that mice which had been immunized with L3 were supplied diffusion chambers containing *D. immitis* third-stage larvae, and the effects on these larvae were used to determine the possible immunity of the mice putatively developed by such immunization. Thus, the papers disclose that implantation of diffusion chambers containing the infectious agent into an immunized animal provides a convenient assessment of the effectiveness of certain directly administered active immunization protocols, but do not describe the use of such chambers to monitor passively transferred protective effects of selected fractions of a target host bloodstream.

Protection against parasitic helminth infections is difficult to achieve because, as heretofore stated, the complexity of the parasitic infection makes the choice of a candidate immunogen for vaccination very difficult. Even naturally conferred immunity cannot be assured to exist, as dogs with previous or existing infections with *D. immitis* can be reinfected (see, for example, Grieve et al., 1983, *Epidemiologic Reviews* 5, p. 220-246) . However, this review also reports that there is some evidence of a naturally occurring protective immune response, which apparently limits the population of mature worms in infected dogs.

Furthermore, it has been possible to induce protective immunity artificially. Wong, et al., 1974, *Exp. Parasitol*. 35, p. 465-474, reported the immunization of dogs with radiation-attenuated infective larvae. The dogs were protected to varying degrees upon challenge. Blair, et al., 1982 in *Fifth International Congress of Parasitology,* Toronto, Canada, reported successful immunization by infecting the dogs and terminating the infection at the fourth larval stage by chemotherapy:

Grieve, 1989, *Proc. Heartworm Symp*., p. 187-190*,* reviewed the status of attempts to produce vaccines against heartworm in dogs. This report summarizes the use of infective larvae implanted in an inert diffusion chamber which permits the influx of cells and/or serum from the host and outflow of parasite material from the chamber to assess the effectiveness of inoculation protocols in both dogs and mice. The use of immunization with infective larvae was demonstrated to be partially effective in protection against subsequent challenge.

An alternative approach to finding, for example, a heartworm vaccine has been to attempt to identify prominent antigens in the infective stage of *D. immitis.* Philipp, et al., 1986, *J. Immunol.* 136, p. 2621-2627, reports a 35-kilodalton (kD) major surface antigen of *D. immitis* third stage larvae which was capable of immunoprecipitation with sera from dogs carrying an occult experimental *D. immitis* infection or with sera from dogs immunized by irradiated third stage larvae. In addition, this group reported (Davis, et al., 1988, Abstract 404, 37th Annual Meeting, *Am. Soc. Trop. Med. Hyg.*) three major surface proteins of the L4 having molecular weights of 150 kD, 52 kD, and 25 kD. The 25 kD molecule seemed unique to L4 larvae.

Ibrahim, et al., 1989, *Parasitol.* 99, p. 89-97, using *D. immitis* L3 larvae labeled with ¹²⁵I, showed that a 35 kD and 6 kD component were shed into the culture medium by developing parasites. They further showed that antibodies from immunized rabbits and infected dogs immunoprecipitated the 35 kD, but not the 6 kD, component.

Scott, et al, 1990, *Acta Tropica* 47, p. 339-353, reported characterization of the surface-associated molecules of *D. immitis* L2, L3, and L4 by radiolabeling techniques and SDS-PAGE (sodium dodecyl sulfate polyacrylamide gel electrophoresis). They found major labeled components of 35 kD and 6 kD in extracts from iodine-labeled L2 and L3; lactoperoxidase-catalyzed labeling revealed components of apparent molecular weights 66 kD, 48 kD, 25 kD, 16.5 kD, and 12 kD. Iodine labeling of surface-associated molecules of L4 gave molecules of apparent molecular weights of 57 kD, 40 kD, 25 kD, 12 kD, and 10 kD; lactoperoxidase-catalyzed labeling showed additional bands of 45 kD, 43 kD, and 3 kD. However, these antigens were identified using uncharacterized serum sources.

Other approaches to obtaining vaccines against parasites in general have focused on the production of neutralizing antibodies. For example, both *in vitro* studies by Tanner, et al., 1981, *Trans. Roy. Soc. Trop. Med. Hyg.* 75, p. 173-174 and by Sim et al., 1982, *Trans. Roy. Soc. Trop. Med. Hyg.* 76, p. 362-370, and *in vivo* studies by Parab et al., 1988, *Immunol.* 64, p. 169-174, have demonstrated that antibodies are effective alone or with other immune components in killing filarial L3 from *Dipetalonema (Acanthocheilonema) viteae* or *Brugia malayi.* Furthermore, passive immunity to *Schistosoma mansoni* has been transferred from immune rats or humans to normal mice (see, for example, Sher, et al., 1975, *Parasitol.* 70, p. 347-357; Jwo et al., 1989, *Am. J. Trop. Med. Hyg.* 41, p. 553-562). None of these studies involved the use of an *in vivo* assay to determine the ability of serum, or cellular, components to be a useful screening tool for identifying protective antigens. Neither has any of these studies yet identified an effective vaccine.

### Summary of the Invention

In one aspect of the present invention, there is provided an isolated parasitic helminth nucleic acid molecule comprising a nucleic acid sequence capable of hybridizing, under stringent conditions, to at least one *Dirofilaria immitis* (*D. immitis*) nucleic acid molecule selected from the group consisting of *D. immitis* nucleic acid sequence and *D. immitis* nucleic acid sequence In one embodiment, the isolated nucleic acid sequence encodes a protein capable of selectively binding to at least one component of immune serum that is capable of inhibiting helminth development. In one embodiment there is provided an isolated parasitic helminth nucleic acid of the present invention, wherein said parasitic helminth is selected from the group consisting of *Dirofilaria, Onchocera, Brugia, Wuchereria, Loa Acanthocheilonema, Dipetalonema, Setaria, Parafilaria* and *Stephanofilaria* filarial nematodes.

In one embodiment, the isolated nucleic acid sequence is selected from the group consisting of *D. immitis* nucleic acid sequence and and complements thereof.

The present invention also includes recombinant molecules and recombinant cells that include isolated nucleic acids of the present invention. In one embodiment, there is provided a recombinant molecule comprising at least one nucleic acid molecule of the present invention operatively linked to at least one transcription control sequence. In another embodiment there is provided a recombinant cell comprising a cell transformed with at least one isolated nucleic acid sequence of the present invention in a manner such that said recombinant cell is capable of expressing said isolated nucleic acid sequence.

Another aspect of the present invention includes an isolated parasitic helminth protein capable of selectively binding to at least one component of immune serum that is capable of inhibiting helminth development, said protein being encoded by a parasitic helminth nucleic acid sequence capable of hybridizing, under stringent conditions, to a nucleic acid sequence complementary to a nucleic acid sequence selected from the group consisting of and

In one embodiment the protein comprises an amino acid sequence selected from the group consisting of and Preferred immune serum is derived from an animal that is immune to infection by the helminth, and preferably from an animal immunized with third stage and/or fourth stage larvae.

Yet another aspect of the present invention is an isolated antibody capable of selectively binding to a parasitic helminth protein, said antibody being produced by a method comprising administering to an animal an effective amount of an isolated protein to produce said antibody, said protein being capable of selectively binding to at least one component of immune serum that is capable of inhibiting helminth development, said protein being encoded by a parasitic helminth nucleic acid sequence capable of hybridizing, under stringent conditions, to a nucleic acid sequence complementary to a nucleic acid sequence selected from the group consisting of and

Yet another aspect of the present invention is a therapeutic composition capable of protecting an animal from parasitic helminth infection when administered to said animal in an effective manner, said composition comprising an isolated parasitic helminth nucleic acid molecule comprising a nucleic acid sequence capable of hybridizing, under stringent conditions, to at least one *Dirofilaria immitis (D. immitis)* nucleic acid molecule selected from the group consisting of *D. immitis* nucleic acid sequence and *D.immitis* nucleic acid sequence

A composition of the invention can include an exipient, adjuvant, and/or carrier. Preferably, the therapeutic composition protects the animal against heartworm.

Preferred parasitic helminths of the present invention include nematodes, cestodes and trematodes, with filarial, ascarid, strongyle and trichostrongyle nematodes being more preferred. Dirofilaria, Onchocerca, Brugia, Wuchereria, Loa, Acanthocheilonema, Dipetalonema, Setaria, Parafilaria and Stephanofilaria filarial nematodes are even more preferred, and D. immitis, the parasite that causes heartworm, is even more preferred.

Preferably the nematode is a filarial nematode, more preferably *D. immitis*. One embodiment of the present invention are antibodies immunoreactive with such proteins.

### Brief Description of the Figures

FIG. 1 depicts a chromatogram of the separation of larval ES by cation exchange chromatography.
FIG. 2 depicts a chromatogram of the separation of tryptic fragments of P22U by C₁₈ reverse phase chromatography; P22U was purified by cation exchange and C₄ reverse phase chromatography.
FIG. 3 shows the hydrophilicity plot and protein characteristics calculated for the sequences of the P20.5 (also referred to as 20 kD) and P22L (also referred to as 22L kD) proteins.

### Detailed Description of the Invention

The present invention includes isolated parasitic helminth proteins and mimetopes thereof that are capable of selectively binding to at least one component of a serum collected from an animal that is immune to infection by the helminth, the serum being capable of inhibiting helminth development; that is, the protein is immunoreactive with a component validated as protective in an immune host using, for example, the method disclosed in Grieve et al., PCT International Publication No. WO 92/13560, published August 20, 1992. The ability of such proteins and mimetopes to selectively bind to components in such a serum is believed to suggest the ability of such proteins and mimetopes to protect an animal from parasite infection when such proteins and/or mimetopes are administered to an animal in an effective manner.

Animals that are immune to infection by parasitic helminths are animals that exhibit an immune response that is sufficient to protect the animal from such infection. Immune animals typically are animals that have been administered larval, adult and/or microfilarial helminths in a manner effective to elicit a protective response, preferably using irradiated helminths or a chemically-abbreviated infection protocol. For example, dogs receiving chemically abbreviated *D. immitis* larval infections exhibit significant immunity to challenge infections. Furthermore, sera obtained from such dogs are effective in passively transferring larval killing and stunting capabilities to mice. Preferred immune animals are those that have been immunized against helminth larvae, particularly against L3 and/or L4 larvae, since, in accordance with the present invention, it is particularly desirable to prevent L3 larvae introduced into an animal from developing into adult parasites. It should be noted, however, that immune animals do not preclude naturally-infected animals that generate protective antibodies.

According to the present invention, an isolated, or biologically pure, parasitic helminth protein, is a protein that has been removed from its natural milieu. As such, "isolated" and "biologically pure" do not necessarily reflect the extent to which the protein has been purified. An isolated parasitic helminth protein can be obtained from its natural source. Alternatively, the isolated parasitic helminth protein can be produced using recombinant DNA technology or chemical synthesis. Isolated proteins include full-length proteins as well as modified versions of the protein in which amino acids have been deleted (e.g., a truncated version of the protein, such as a peptide), inserted, inverted, substituted and/or derivatized (e.g., glycosylated, phosphorylated, acetylated) such that the modified version of the protein has a biological function substantially similar to that of the natural protein (i.e., functionally equivalent to the natural protein). Modifications can be accomplished by techniques known in the art including, but not limited to, direct modifications to the protein or modifications to the gene encoding the protein using, for example, classic or recombinant DNA techniques to effect random or targeted mutagenesis. Isolated proteins of the present invention, including modified versions thereof, can be identified in a straight-forward manner by the proteins' ability to selectively bind to at least one component of anti-parasitic helminth immune serum. As used herein, immune serum refers to serum that is capable of inhibiting helminth development that preferably is derived (e.g., obtained from) an animal that is immune to the helminth. The minimum size of isolated proteins of the present invention is sufficient to form an epitope, a size that is typically at least about 7 to about 9 amino acids. As is appreciated by those skilled in the art, an epitope can include amino acids that naturally are contiguous to each other as well as amino acids that, due to the tertiary structure of the natural protein, are in sufficiently close proximity to form an epitope.

In accordance with the present invention, a mimetope refers to any compound that is able to mimic the ability of an isolated protein of the present invention to selectively bind to at least one component of anti-parasitic helminth immune serum. A mimetope can be a peptide that has been modified to decrease its susceptibility to degradation but that still retains its selective binding ability. Other examples of mimetopes include, but are not limited to, anti-idiotypic antibodies, or fragments thereof, that include at least one binding site that mimics one or more epitopes of an isolated protein; non-proteinaceous immunogenic portions of an isolated protein (e.g., carbohydrate structures); and synthetic or natural organic molecules, including nucleic acids, that have a structure similar to at least one epitope of an isolated protein of the present invention. Such mimetopes can be obtained, for example, by affinity chromatography techniques using immune sera of the present invention or antibodies raised against isolated proteins of the present invention.

As used herein, the term "selectively binds to" refers to the ability of isolated proteins and mimetopes thereof to bind to serum collected from animals that have been exposed to parasitic helminths (either through natural infection or through administration of helminths) but essentially not to bind, according to standard detection techniques (such as those described in Sambrook et al., *Molecular Cloning: A Laboratory Manual*, Cold Spring Harbor Labs Press, 1989) to serum collected from animals that have not been exposed to parasitic helminths (i.e., naive animals). Preferably, the isolated proteins and mimetopes are able to bind to anti-parasitic helminth immune serum with high affinity. The ability of a protein or mimetope thereof to selectively bind to anti-parasitic helminth immune serum can be measured using a variety of methods known to those skilled in the art including immunoblot assays, immunoprecipitation assays, enzyme immunoassays (e.g., ELISA), radioimmunoassays, immunofluorescent antibody assays and immunoelectron microscopy. It should be noted that the ability of an isolated protein or mimetope thereof to selectively bind to immune serum raised against a certain stage of helminth development does not preclude the isolated protein or mimetope from being able to also bind to immune serum raised against other stages of helminth development. For example, the ability of an isolated protein or mimetope thereof to selectively bind to an anti-larval immune serum does not preclude the isolated protein or mimetope from being able to also bind to anti-microfilarial and/or anti-adult immune serum.

As used herein, the phrases "capable of selectively binding to at least one component of a serum collected from an animal that is immune to infection by the helminth, the serum being capable of inhibiting helminth development" and "specifically immunoreactive with validated components of immune host serum or tissue" have similar meanings. "Validated components" are components which have been shown in the method of the invention, as described herein, to exert a deleterious effect on the parasitic nematodes when supplied in a diffusion chamber to a neutral host which has been administered the component. By "specifically immunoreactive" is meant that the immunogen is capable of binding the validated component as derived from an immune susceptible host, but is incapable of binding components found in nonimmune counterparts in this species. By "susceptible host " is meant a host species that is ordinarily susceptible to infestation by the nematode parasite in question. Individual members of the susceptible host species may have acquired immunity to this infestation.

One embodiment of the present invention is the use of anti-parasitic helminth immune serum to identify isolated proteins and mimetopes of the present invention, a technique referred to herein as immune serum screening assay. Immune serum can be raised against a parasitic helminth by administering the helminth to an animal under conditions that elicit an immune response. Immune serum can be raised against larval, microfilarial, and/or adult helminths, preferably against larvae, and more preferably against L3 and/or L4 larvae. Immune sera of the present invention are capable not only of inhibiting development of the species of helminth that elicited the immune response, but also of helminth species that immunologically cross-react with the immune sera. Due to the similarity between helminths, immune sera of the present invention are capable of reacting with a large variety of helminths. Inhibiting the development of helminths includes killing, reducing the growth of, blocking the maturation of, altering the morphology of, altering the metabolism of, and/or otherwise being detrimental to the helminth.

Any animal that is capable of mounting an immune response to protect itself from helminth infection is a suitable animal to which helminths can be administered and from which immune serum can be collected. For example, a preferred animal from which to collect serum capable of inhibiting the development of *D. immitis* is a dog that has been administered L3 and/or L4 *D. immitis* larvae under conditions that elicit an immune response.

The ability of immune serum of the present invention to inhibit parasitic helminth development can be determined in a number of ways. A preferred method to monitor the ability of immune serum to inhibit the development of an infectious agent is disclosed by Grieve et al., WO 92/13560, *ibid.* As disclosed therein, for example, the ability of an anti-parasitic helminth larval immune serum to inhibit larval development can be determined as follows. Briefly, a naive animal (i.e., an animal not previously exposed to parasitic helminth larvae) is implanted with at least one diffusion chamber containing helminth larvae, preferably L3 larvae. The animal is also administered either the anti-larval immune serum to be tested or a control non-immune serum, preferably at a site near the diffusion chambers. After a suitable period of time, for example, from about three to about four weeks for *D. immitis* larvae implanted in mice, the diffusion chambers are removed, and the effects of the immune serum on larval growth and development are determined by, for example, comparing larval growth and survival in chambers exposed to anti-larval immune serum with the growth and survival of larvae in diffusion chambers exposed to non-immune serum. A significant number of larvae exposed to anti-larval immune serum are either killed or stunted compared to larvae exposed to non-immune serum.

Grieve et al., WO 92/13560, *ibid.*, further discloses use of the immune serum screening assay to screen for, and hence identify, desired proteins that selectively bind to the immune serum. Briefly, the immune serum can be contacted with a protein-containing composition under conditions that permit selective binding by desired proteins to components in the serum. Complexes between the proteins and serum components are recovered, the proteins are separated from the serum components and are then analyzed. Nucleic acid sequence encoding such proteins can be identified using known recombinant DNA techniques, such as those described in Sambrook at al., *ibid.* In another embodiment, the immune serum screening assay can be used to identify nucleic acid sequences encoding isolated proteins of the present invention by screening parasite helminth expression cDNA libraries with immune sera of the present invention to identify proteins expressed by individual clones that are capable of selectively binding to the immune sera. The immune serum screening assay can also be used to identify mimetopes capable of selectively binding to immune serum, such as to anti-L3 and/or L4 larval immune serum. Mimetopes can also be designed or improved using information derived from proteins identified by the immune serum screening assay. It should be appreciated that not only serum, but also other immunogenic components of bodily fluids collected from animals immune to helminth infection, such as cells, specific antibodies, and fragments thereof, can be used in the immune serum screening assay.

As disclosed in Grieve et al., WO 92/13560, *ibid*., anti-larval immune serum has been used to identify nematode proteins expressed during L3 and/or L4 that have molecular weights of 66 kD, 65 kD, 59 kD, 39 kD, 33 kD, 23/24 kD, 22/20.5 kD and 14 kD, as determined by their migration patterns when submitted to Tris-glycine SDS PAGE. Nucleic acid sequences encoding these proteins can be identified using anti-L3 and/or L4 larval immune serum to screen larval nematode cDNA expression libraries.

One embodiment of the present invention is an isolated parasitic helminth nucleic acid sequence that encodes an isolated protein of the present invention. As used herein, an isolated parasitic helminth nucleic acid sequence is a nucleic acid sequence that has been removed from its natural milieu. As such, "isolated" does not reflect the extent to which the nucleic acid sequence has been purified. An isolated nucleic acid sequence can be DNA, RNA, or derivatives of either DNA or RNA. Isolated nucleic acid sequences of the present invention include sequences that encode at least one epitope capable of selectively binding to immune sera of the present invention as well as oligonucleotides that can function in a variety of ways, including, but not limited to, as probes, primers, and therapeutic agents using, for example, antisense-, triplex formation- and/or ribozyme-based technologies. An isolated parasitic helminth nucleic acid sequence can be obtained from its natural source either as an entire gene or a portion thereof, the minimal size of a portion being a size that can form a stable hybrid with a similar nucleic acid sequence under stringent conditions. As such, isolated nucleic acid sequences can include regulatory regions that control expression of the corresponding coding region (e.g., transcription or translation control regions or control sequences), full-length or partial coding regions, and combinations thereof. Isolated parasitic helminth nucleic acid sequences can also be produced using recombinant DNA technology (e.g., PCR amplification, cloning) or chemical synthesis. Isolated parasitic helminth nucleic acid sequences include functional equivalents of natural sequences, including, but not limited to, natural allelic variants and modified nucleic acid sequences in which nucleotides have been inserted, deleted, substituted, and/or inverted in such a manner that such modifications do not substantially interfere with the nucleic acid sequence's ability to encode an epitope recognized by immune sera of the present invention or do not substantially interfere with the ability of the nucleic acid sequence to form stable hybrids under stringent conditions with natural isolates. As used herein, stringent hybridization conditions refer to standard hybridization conditions under which nucleic acid sequences, including oligonucleotides, are used to identify similar sequences. Such standard conditions are disclosed, for example, in Sambrook et al., *ibid.* Examples of such conditions are provided in the Examples section.

Functionally equivalent nucleic acid sequences can be obtained using methods known to those skilled in the art (see, for example, Sambrook et al., *ibid*.). For example, nucleic acid sequences can be modified using a variety of techniques including, but not limited to, classic mutagenesis techniques and recombinant DNA techniques, such as site-directed mutagenesis, chemical treatment of a nucleic acid to induce mutations, restriction enzyme cleavage of a nucleic acid fragment, ligation of nucleic acid fragments, polymerase chain reaction (PCR) amplification and/or mutagenesis of selected regions of a nucleic acid sequence, synthesis of oligonucleotide mixtures and ligation of mixture groups to "build" a mixture of nucleic acid sequences, and combinations thereof. Functionally equivalent nucleic acids can be selected from a mixture of modified nucleic acid sequences by screening for the function of the protein encoded by the nucleic acid sequence (e.g., ability to bind to immune serum) and/or by hybridization with natural nucleic acid sequences under stringent conditions.

Due to the similarity between parasitic helminth genomes, isolated proteins and corresponding nucleic acid sequences of the present invention can be from any parasitic helminth. Preferred helminths include nematode, cestode and trematode parasites. More preferred helminths include filarial, ascarid, strongyle and trichostrongyle nematodes. Even more preferred helminths include *Dirofilaria, Onchocerca, Brugia, Wuchereria, Loa, Acanthocheilonema, Dipetalonema, Setaria, Parafilaria* and *Stephanofilaria* filarial nematodes. A particularly preferred parasitic helminth of the present invention is *D. immitis*, the filarial nematode that causes heartworm.

Other suitable parasitic nematodes include *Dipetalonema perstans, Dipetalonema streptocerca, Wuchereria bancrofti, B. malayi, Mansonella ozzardi, Loa loa, O. volvulus, Strongyloides spp., Strongylus spp., Haemonchus spp., Trichostrongylus spp., Ostertagia spp., Cooperia spp., Dictyocaulus spp., Nematodirus spp.,* Cyathostominae (small strongyles of horses), *Oesophagostomum spp., Chabertia ovina, Ancylostoma spp., Uncinaria spp., Bunostomum spp., Filaroides spp., Aelurostrongylus abstrusus,* those nematodes of the order Ascaridida (Ascarids), *Trichinella spiralis, Trichuris spp., Angiostrongylus spp.* and *Enterobius vermicularis.* *D. immitis* nucleic acid sequence p4, also referred to as *D. immitis* p4, is a nucleic acid sequence of about 913 nucleotides in length that has been isolated from a *D. immitis* L3 and/or L4 cDNA expression library using immune serum collected from a dog that was immunized by repeated chemically abbreviated infections (e.g., infect with about 200 L3, wait about 60 days, treat with ivermectin, wait about 60 days, reinfect, etc.).Genomic sequences including p4 coding sequences also apparently include about 710 nucleotides comprising one or more introns.

Sequencing of *D. immitis* p4 cDNA has resulted in the nucleic acid sequence disclosed in SEQ ID No:1. It should be noted that sequencing technology is not entirely errorfree and that SEQ ID NO:1, as such, represents an apparent nucleic acid sequence of *D. immitis* p4. The deduced translation of SEQ ID NO:1, represented in SEQ ID NO:2, suggests that *D. immitis* p4 comprises an open reading frame of about 303 amino acids and, as such, represents only a portion of the entire coding sequence of the gene. The nucleic acid contained in *D. immitis* p4, however, is sufficient to encode a protein that selectively binds with anti-*D. immitis* larval immune serum, as demonstrated by the manner in which the nucleic acid sequence was isolated. The deduced translation of SEQ ID NO:1 suggests that the protein encoded by *D. immitis* p4 has a molecular weight of about 35.5 kilodaltons (kD) and an estimated pI of 4.26.

The protein encoded by *D. immitis* p4 is further characterized by having an LDL receptor-related protein (LDLr) class A cysteine-rich motif of about 9 amino acids that is also found in several other proteins, including mammalian low density lipoprotein (LDL) receptors, LDL receptor-related proteins, human and mouse alpha-2-macroglobulin receptors and rat renal GP 330 glycoprotein. Each of these proteins, including *D. immitis* P4, share the sequence DDCGDGSDE (i.e., Aspartic Acid -- Aspartic Acid -- Cysteine -- Glycine -- Aspartic Acid -- Glycine -- Serine -- Aspartic Acid -- Glutamic Acid). A conserved stretch of eight of the nine amino acids is also found in the free-living (i.e., non-parasitic) nematode *Caenorhabditis elegans* LDL receptor-related protein and *C. elegans* basement membrane proteoglycan. This LDLr class A, cysteine-rich motif is likely to be conserved in proteins encoded by p4-related sequences of other helminths (i.e., nucleic acid sequences that hybridize under stringent conditions with *D. immitis* p4). As such, p4-related nucleic acid sequences may be identified using oligonucleotide probes that encode such LDLr class A motifs. Furthermore, the LDLr class A motif in P4-related proteins represents a target for development of therapeutic compositions to protect animals from parasitic helminth infection, as discussed below.

Due to the similarities between parasitic helminths, as heretofore disclosed, one can use *D. immitis* p4 sequences to obtain other parasitic helminth nucleic acid sequences that are capable of hybridizing, under stringent conditions, to at least a portion of *D. immitis* p4. Preferred helminths are heretofore disclosed.

Knowing the sequence of *D. immitis* p4 allows one skilled in the art to make copies of the sequence as well as to obtain nucleic acid sequences including *D. immitis* p4 and nucleic acid sequences that contain fragments of *D. immitis* p4. As such, examples of isolated nucleic acid sequences include SEQ ID NO:1 or a functional equivalent thereof, a nucleic acid sequence containing at least a portion of SEQ ID NO:1 or a functional equivalent thereof, and a fragment of SEQ ID NO:1 or a functional equivalent thereof, assuming the accuracy of SEQ ID NO:1. Another *D. immitis* nucleic acid sequence is p22U. A protein encoded by such a nucleic acid sequence is preferably capable of selectively binding to at least one component of anti-parasitic helminth immune serum, and more *D. immitis* nucleic acid sequence p22U, also referred to as *D. immitis* p22U, encodes at least a substantial portion of a basic *D. immitis* protein, referred to as *D. immitis* P22U protein, that migrates at an apparent molecular weight of about 22 kD when submitted to Tris-glycine SDS (sodium dodecyl sulfate) PAGE (polyacrylamide gel electrophoresis). *D. immitis* P22U protein has been identified in *larval* ES (excretory-secretory) extracts as well as in extracts of L3, L4 and adults. *D. immitis* p22U is about 1016 nucleotides in length. Sequencing of *D. immitis* p22U has resulted in the nucleic acid sequence disclosed in SEQ ID NO:3. It should be noted that sequencing technology is not entirely error-free and that SEQ ID NO:3, as such, represents an apparent nucleic acid sequence of *D. immitis* p22U. The deduced translation of SEQ ID NO:3, represented in SEQ ID NO:4, suggests that *D. immitis* p22U includes an open reading frame of about 208 amino acids followed by a stop codon. The translation start site is as yet unknown although there are two "in-frame" potential start codons at about amino acid 13 and about amino acid 19 of corresponding (i.e., deduced) amino acid sequence SEQ ID NO:4.The deduced amino acid sequence suggests a protein having a molecular weight of about 22 kD and an estimated pI of about 9.6.

*D. immitis* p22U can be isolated in a number of ways including, but not limited to, screening an L3, L4, or adult expression cDNA library with appropriate immune serum or with antibodies raised against *D. immitis* P22U protein. Alternatively, amino acid sequence information can be derived from purified *D. immitis* P22U protein that can be used to design oligonucleotide probes and/or primers that can be used to screen and/or amplify sequences from an appropriate cDNA or genomic library.

Due to the similarities between parasitic helminths, as heretofore disclosed, one can use *D. immitis* p22U to obtain other parasitic helminth nucleic acid sequences that are capable of hybridizing, under stringent conditions, to at least a portion of *D. immitis* p22U. Preferred helminths are heretofore disclosed.

Knowing the sequence of *D. immitis* p22U allows one skilled in the art to make copies of the sequence as well as to obtain nucleic acid sequences including *D. immitis* p22U and nucleic acid sequences that contain fragments of *D. immitis* p22U. As such, examples of isolated nucleic acid sequences include SEQ ID NO:3 or a functional. equivalent thereof, a nucleic acid sequence containing at least a portion of SEQ ID NO:3 or a functional equivalent thereof, and a fragment of SEQ ID NO:3 or a functional equivalent thereof, assuming the accuracy of SEQ ID NO:3.

The present invention includes oligonucleotides that are capable of hybridizing, under stringent conditions, to complementary regions of other, preferably longer, nucleic acid sequences of the present invention. The oligonucleotides can be RNA, DNA, or derivatives of either. The minimal size of such oligonucleotides is the size required to form a stable hybrid between a given oligonucleotide and the complementary sequence on another nucleic acid sequence of the present invention. As such, the size is dependent on nucleic acid composition and percent homology between the oligonucleotide and complementary sequence as well as upon hybridization conditions per se (e.g., temperature, salt concentration). For AT-rich nucleic acid sequences, such as those of *D. immitis*, oligonucleotides typically are at least about 15 to about 17 bases in length. The size of the oligonucleotide must also be sufficient for the use of the oligonucleotide in accordance with the present invention. Oligonucleotides of the present invention can be used in a variety of applications including, but not limited to, as probes to identify additional nucleic acid sequences, as primers to amplify or extend nucleic acid sequences, or in therapeutic applications to inhibit, for example, expression of nucleic acid sequences into parasitic helminth proteins that are important in the life cycle of the parasite. Such therapeutic applications include the use of such oligonucleotides in, for example, antisense-, triplex formation-, and/or ribozyme-based technologies.

For example, antisense oligonucleotides are generally designed as complements to the messenger RNA encoding the desired protein. The complement binds through Watson-Crick base-pairing to the mRNA interfering with translation either by enhancing mRNA degradation by RNAse H, by preventing or inhibiting processing to mature RNA, or by preventing translation. The oligonucleotide may bind either to the translated region or to control sequences in the mRNA.

Similarly, as the transcription of DNA involves partial disassembly of the double helix, antisense oligonucleotides may also bind to transcribed or nontranscribed regions of the DNA to inhibit transcription. Absolute homology between the target and. the antisense sequences is preferred but not required for the inhibition. Holt, J.T. et al., *Proc Natl Acad Sci* (1986) 83:4794.

Oligonucleotides may also be designed to form a triplex DNA structure with the intact duplex gene according to certain binding rules. Moffat, A.S., *Science* (1991) 252:1374-1375. When this triplex structure is formed in the promoter region of a gene, it has been shown to disrupt transcription of that gene. Orson, F.M. et al., *Nuc Acids Res* (1991) 19:3435-3441. Again, the oligomer designed to form a triplex can be designed to bind the duplex gene in either regulatory or transcribed regions or both.

The invention, therefore, also includes methods to interfere with the production of parasitic helminth proteins by use of the antisense or triple helix-forming techniques. The relevant oligomers may be administered to a host harboring the parasite in order to effect this control.

Isolated nucleic acid sequences of the present invention can be obtained in a variety of ways. For example, an isolated nucleic acid sequence of the present invention can be obtained by a method that includes induction of a L3 and/or L4 expression library under conditions that promote production of larval proteins encoded by the library; contacting the library with immune serum collected from an animal that is immune to infection by L3 and/or L4; and selecting a colony or phage plaque that contains a nucleic acid sequence encoding a protein capable of selectively binding to the serum. Conventional culturing and selection methods are taught, for example, in Sambrook et al., ibid. An example of this methodology is also provided in the Examples section.

As a comparative example, an isolated nucleic acid sequence is obtained by a method including contacting, under stringent hybridization conditions, at least one oligonucleotide with a parasitic helminth cDNA library, such that the oligonucleotide includes nucleic acid sequences that encode at least a portion of *D. immitis* P4 and/or *D. immitis* P22U; and selecting a colony or phage plaque having a nucleic acid sequence that hybridizes under stringent conditions with the oligonucleotide. Alternatively, oligonucleotide primers, including nucleic acid sequences that encode at least portions of *D. immitis* P4 and/or *D. immitis* P22U, can be used to amplify, by polymerase chain reaction (PCR) amplification, nucleic acid sequences that include at least a portion of *D. immitis* p4 and/or *D. immitis* p22U. An example of these methodologies is provided in the Examples section.

As a comparative example, an isolated nucleic acid sequence is obtained by a method including contacting a collection of nucleic acid sequences, such as a parasitic helminth cDNA library, with *D. immitis* p4 or a portion thereof, or with *D. immitis* p22U or a portion thereof, under stringent hybridization conditions: and identifying a nucleic acid sequence that hybridizes to either *D. immitis* p4 or the portion thereof, or with *D. immitis* p22U or the portion thereof, under such conditions. Such a technique can be used to clone a nucleic acid sequence using standard hybridization techniques or to amplify a nucleic acid sequence using PCR amplification. Alternatively, serum raised against *D. immitis* P4 or *D. immitis* P22U could be used to screen cDNA expression libraries.

The present invention also includes immunogens which are isolatable from (i.e. , can be isolated from) the L3 and L4 larval stages of nematodes parasitic in mammals which have molecular weights of about 22 kD, or about 20.5 kd and which can be identified using the method disclosed in Grieve et al., WO/92/13560, *ibid.* The invention is also directed to corresponding recombinant aspects of these proteins and to immunogenic fragments of the proteins and their recombinant aspects.

This embodiment of the invention is illustrated by the identification of 39 kD, 22 kD and 20.5 kD immunogens from *D. immitis*, using serum validated by the invention method. The retrieval of genes encoding the immunogens provides suitable probes for retrieval of genes encoding the corresponding proteins in related nematodes capable of parasitism in mammalian hosts. Thus, the invention is directed to the illustrated. 22 kD, and 20.5 kD *D. immitis* proteins and their related counterparts which are encoded by DNAs capable of hybridizing under standard conditions to the DNA encoding the respective illustrated *D. immitis* protein. As such, the present application includes *, D. immitis* P22L, *D. immitis* P20.5, additional parasitic helminth proteins sharing significant homology with *D. immitis* P22L, or *D. immitis* P20.5, nucleic acid sequences encoding any of these proteins, mimetopes of any of these proteins, and antibodies that selectively bind to any of these proteins, as well as uses of these proteins, mimetopes, nucleic acid sequences, and antibodies.

The terms "39 kD protein" and "P39" each refer to parasitic helminth proteins of this apparent molecular weight, as determined by Tris-glycine SDS-PAGE, isolatable from the L3 or L4 larval stage of *D. immitis* or from related nematodes, or other helminths, which are parasitic in mammals. The 39 kD protein isolatable from *D. immitis* has the amino acid sequences shown hereinbelow. Although the calculated molecular weight of the *D. immitis* protein is only 39,820, apparent molecular weights may be different, depending on the particular system of measurement used. In particular, P39 migration in Tris-glycine SDS-PAGE indicates a molecular weight of about 39 kD.

The term "22/20.5 kD protein" refers to parasitic helminth proteins of these apparent molecular weights isolatable from the L3 or L4 larval stage of *D. immitis* or from related nematodes, or other helminths, which are parasitic in mammals. The amino acid sequences of the 22/20.5 kD proteins isolatable from *D. immitis* are disclosed below. As shown, the 20.5 kD protein is the cleavage product of the 22 kD protein wherein 21 amino acids of a leader sequence are deleted to yield the smaller protein. Although the calculated molecular weights of the *D. immitis* proteins are only 17.5 kD for the larger and 15.3 kD for the smaller protein, apparent molecular weights may be higher, depending on the particular system of measurement used. The 22 kD and 20.5 kD proteins are also referred to herein as P22L and P20.5, respectively. P22L is the lower, and P22U is the upper, of two bands that migrate at about 22 kD, as separated Tris-glycine SDS PAGE and a molecular weight of about 19 kD as measured by Tris-tricine SDS-PAGE. P20.5 has a molecular weight of about 20.5 kD as measured by Tris-glycine SDS-PAGE and of about 16 kD as measured by Tris-tricine SDS-PAGE.

P39, P22L and P20.5 from *D. immitis* are specifically immunoreactive with components of immune dog serum which have been validated as protective by the invention method. Similarly, corresponding proteins in other nematode, and other helminth, species parasitic in mammals are specifically immunoreactive with protective components of immune members of the relevant mammalian species, validated by the invention method. The present invention, therefore, provides access to a group of proteins which are contained in the L3 or L4 larval stages of nematodes which are parasitic in mammals and which are useful in preparation of vaccines protective against these parasites.

The invention provides not only P22L and P20.5, but also the parasitic helminth nucleic acid sequences encoding the respective proteins. Availability of these nucleic acid sequences permits retrieval of homologous proteins from related nematode and other helminth species. The nucleic acid sequences, or portions thereof, can be used as probes under conditions of standard stringency (i.e., under stringent conditions as defined herein), depending on the library and length of probe, to isolate DNA encoding corresponding proteins in these related species. Further, antibodies immunoreactive with P39 or with P22L and P20.5 of *D. immitis* may be used as screening tools to identify nucleic acid sequences encoding such proteins in expression libraries prepared from related species. *D. immitis* nucleic acid sequence p39, also referred to as *D. immitis* p39, is a nucleic acid sequence of about 1100 to about 1200 nucleotides in length that has been isolated from a *D. immitis* L3 and/or L4 cDNA expression library using immune serum collected from a dog that was immunized as heretofore disclosed. Genomic sequences including p39 coding sequences also apparently include about 1280 nucleotides comprising one or more introns.

Sequencing of *D. immitis* p39 cDNA isolates has resulted in the following deduced nucleic acid sequence of the apparent coding region (additional flanking sequences are presented in the Examples): It should be noted that sequencing technology is not entirely error-free and that the above nucleic acid sequence, as such, represents an apparent nucleic acid sequence of *D. immitis* p39. The deduced translation of the nucleic acid sequence, namely a deduced *D. immitis* P39 amino acid sequence of about 300 amino acids, as follows:

One embodiment of the present invention is an isolated parasitic helminth nucleic acid sequence that is capable of hybridizing, under stringent conditions, to at least a portion of *D. immitis* nucleic acid sequence p22L. A protein encoded by such a nucleic acid sequence is preferably capable of selectively binding to at least one component of anti-parasitic helminth immune serum, and more preferably to anti-L3 and/or L4 larval immune serum. *D. immitis* nucleic acid sequence p22L, also referred to as *D. immitis* p22L, is a nucleic acid sequence of about 453 nucleotides in length that has been isolated from a *D. immitis* L3 and/or L4 cDNA expression library using immune serum collected from a dog that was immunized as heretofore disclosed.

Sequencing of a *D. immitis* p22L cDNA has resulted in the following nucleic acid sequence of the apparent coding region: It should be noted that sequencing technology is not entirely error-free and that the above nucleic acid sequence, as such, represents an apparent nucleic acid sequence of *D. immitis* p22L. The deduced translation of the nucleic acid sequence, namely a deduced *D. immitis* P22L amino acid sequence of about 150 amino acids, as follows:

The C-terminal half of P22L is further characterized by sharing at least some amino acid sequence homology with a variety of phospholipase A₂ (PLA2) amino acid sequences, the similarities being particularly well conserved with respect to cysteines and the amino acids comprising the active site. A BLAST search of the NCBI non-redundant data library (SWISS-PROT ver. 23.0, PIR ver. 34.0, GenPept CDS translations from GenBank release 73.1) using amino acids 80-104 (DGKMK HCKTH EACYD QREPQ SWCIL) of the deduced P22L amino acid sequence yielded 40 records, 39 of which were PLA2 sequences. Twenty-five of the 29 SWISS-PLOT match sequences represent PLA2 venoms from a variety of snakes while the other 4 sequences were mammalian pancreatic PLA2 sequences. No non-mammal, non-arthropod eukaryotic entries were found.

PLA2 catalyzes the hydrolysis of the 2-acyl ester group of sn-3-glycerophospholipids. Potential roles of a PLA2 activity in parasites include lipid metabolism; membrane synthesis, remodeling and/or separation (e.g., as part of the molting process); and/or in migration (e.g., PLA2 could aid in disrupting host cell membranes during the tissue migration that occurs during L4). As such, the finding of homologous sequences between the C-terminal portion of P22L (and, inherently, P20.5) and PLA2 suggest the targeting of such sequences in the development of anti-parasite therapeutics that block PLA2 activity, thereby protecting animals from parasite helminth infections.

Yet another embodiment of the present invention is an isolated parasitic helminth nucleic acid sequence that is capable of hybridizing, under stringent conditions, to at least a portion of *D. immitis* nucleic acid sequence p20.5. A protein encoded by such a nucleic acid sequence is preferably capable of selectively binding to at least one component of anti-parasitic helminth immune serum, and more preferably to anti-L3 and/or L4 larval immune serum. *D. immitis* nucleic acid sequence p20.5, also referred to as *D. immitis* p20.5, is a nucleic acid sequence of about 390 nucleotides in length that has been isolated from a *D. immitis* L3 and/or L4 cDNA expression library using immune serum collected from a dog that was immunized as heretofore disclosed. Sequencing of *D. immitis* p20.5 has resulted in the following nucleic acid sequence of the apparent coding region: It should be noted that sequencing technology is not entirely error-free and that the above nucleic acid sequence, as such, represents an apparent nucleic acid sequence of *D. immitis* p20.5. The deduced translation of the nucleic acid sequence, namely a deduced *D. immitis* P20.5 amino acid sequence of about 129 amino acids, as follows:

As disclosed for nucleic acid sequences p4 and p22U, nucleic acid sequences of the present invention encoding P39 (i.e., p39), P22L (i.e., p22L) and L20.5 (i.e., p20.5) include allelic variants and fragments thereof, including oligonucleotides having a variety of uses. Methods to isolate and use nucleic acid sequences p39, p22L and p20.5 as well as the proteins encoded by them are similar to those used in the isolation and use of p4 and p22U. Selected examples are disclosed in the examples section.

The present invention also includes recombinant vectors, which include a parasitic helminth nucleic acid sequence of the present invention inserted into any vector capable of delivering the nucleic acid into a host cell. The vector contains heterologous nucleic acid sequences, that is nucleic acid sequences that are not naturally found adjacent to parasitic helminth nucleic acid sequences of the present invention and that preferably are derived from a species other than the species from which the parasitic helminth nucleic acid sequences are derived. The vector can be either RNA or DNA, either prokaryotic or eukaryotic, and typically is a virus or a plasmid. Recombinant vectors can be used in the cloning, sequencing, and/or otherwise manipulating of nucleic acid sequences of the present invention. One type of recombinant vector, herein referred to as a recombinant molecule and described in more detail below, can be used in the expression of nucleic acid sequences of the present invention. Preferred recombinant vectors are capable of replicating in the transformed cell. Preferred nucleic acid sequences to include in recombinant vectors of the present invention include parasitic helminth nucleic acid sequences capable of hybridizing, under stringent conditions, to at least a portion of *D. immitis* nucleic acid sequence p22L, or to least a portion of *D. immitis* nucleic acid sequence p20.5.

Particularly preferred nucleic acid sequences to included in recombinant vector include *D. immitis* nucleic acid sequence p22L, nucleic acid sequences including D. immitis p22 L, nucleic acid sequence comprising fragments of *D. immitis* p22L, *D. immitis* nucleic acid sequence p20.5, nucleic acid sequences including *D. immitis* p20.5, and nucleic acid sequence comprising fragments of *D. immitis* p20.5.

Isolated proteins of the present invention can be produced in a variety of ways, including production and recovery of natural proteins, production and recovery of recombinant proteins, and chemical synthesis. In one embodiment, an isolated protein of the present invention is produced by culturing a cell capable of expressing the protein under conditions effective to produce said protein, and recovering the protein. A preferred cell to culture is a recombinant cell that is capable of expressing the protein, the recombinant cell being produced by transforming a host cell with one or more nucleic acid sequences of the present invention. Transformation of a nucleic acid sequence into a host cell can be accomplished by any method by which a nucleic acid sequence can be inserted into a cell. Transformation techniques include, but are not limited to, transfection, electroporation, microinjection, lipofection, adsorption, and protoplast fusion. A recombinant cell may remain unicellular or may grow into a tissue, organ or a multicellular organism. Transformed nucleic acid sequences of the present invention can remain extrachromosomal or can integrate into one or more sites within a chromosome of a host cell in such a manner that their ability to be expressed is retained. Preferred nucleic acid sequences with which to transform cells include parasitic helminth nucleic acid sequences capable of hybridizing, under stringent conditions, to at least a portion of *D. immitis* nucleic acid sequence p22L, or to least a portion of *D. immitis* nucleic acid sequence p20.5. Particularly preferred nucleic acid sequences with which to transform cells include *D. immitis* nucleic acid sequence p22L, nucleic acid sequences including *D. immitis* p22L, nucleic acid sequence comprising fragments of *D. immitis* p22L, *D. immitis* nucleic acid sequence p20.5, nucleic acid sequences including *D. immitis* p20.5, and nucleic acid sequence comprising fragments of *D. immitis* p20.5.

Suitable host cells to transform include any cell that can be transformed. Host cells can be either untransformed cells or cells that are already transformed with at least one nucleic acid sequence. Host cells of the present invention either can be endogenously (i.e., naturally) capable of producing isolated proteins of the present invention or can be capable of producing such proteins after being transformed with at least one nucleic acid sequence of the present invention. Host cells of the present invention can be any cell capable of producing an isolated protein of the present invention, including bacterial, yeast, other fungal, insect, animal, and plant cells. Preferred host cells include bacterial, mycobacterial, yeast, insect and mammalian cells, and more preferred host cells include *Salmonella, Escherichia, Bacillus, Saccharomyces, Spodoptera, Mycobacteria, Trichoplusia,* BHK (baby hamster kidney) cells, MDCK cells (normal dog kidney cell line for canine herpesvirus cultivation), CRFK cells (normal cat kidney cell line for feline herpesvirus cultivation) and COS cells. Particularly preferred host cells are *Escherichia coli,* including *E. coli* K-12 derivatives; *Salmonella typhi; Salmonella typhimurium*, including attenuated strains such as UK-1 ᵥ3987 and SR-11 ᵥ4072; *Spodoptera frugiperda; Trichoplusia ni*; MDCK cells; and CRFK cells.

Additional appropriate mammalian cell hosts include other kidney cell lines (e.g., CV-1 monkey kidney cell lines), other fibroblast cell lines (e.g., human, murine or chicken embryo fibroblast cell lines), Chinese hamster ovary (CHO) cells, HeLa cells, mouse NIH/3T3 and/or LMTX³¹ cells. Alternatively, the proteins may be expressed as heterologous protein in myeloma cell lines employing immunoglobulin promoters.

A recombinant cell is preferably produced by transforming a host cell with one or more recombinant molecules, each comprising one or more nucleic acid sequences of the present invention operatively linked to an expression vector containing one or more transcription control sequences. A cell can be transformed with one or more recombinant molecules. The phrase operatively linked, or operably linked, refers to insertion of a nucleic acid sequence into an expression vector in a manner such that the sequence is able to be expressed when transformed into a host cell. As used herein, an expression vector is a DNA or RNA vector that is capable of transforming a host cell, of replicating within the host cell, and of effecting expression of a specified nucleic acid sequence. Expression vectors can be either prokaryotic or eukaryotic, and are typically viruses or plasmids. Expression vectors of the present invention include any vectors that function (i.e.,direct gene expression) in recombinant cells of the present invention, including in bacterial, yeast, other fungal, insect, animal, and plant cells. Preferred expression vectors of the present invention can direct gene expression in bacterial, yeast, insect and mammalian cells and more preferably in the cell types heretofore disclosed.

Expression vectors of the present invention may also contain secretory signals to enable an expressed parasitic helminth protein to be secreted from its host cell or may contain fusion sequences which lead to the expression of inserted nucleic acid sequences of the present invention as fusion proteins. Eukaryotic recombinant molecules may include intervening and/or untranslated sequences surrounding and/or within parasitic helminth nucleic acid sequences.

Nucleic acid sequences of the present invention can be operatively linked to expression vectors containing regulatory sequences such as promoters, operators, repressors, enhancers, termination sequences, origins of replication, and other regulatory sequences that are compatible with the host cell and that control the expression of the nucleic acid sequences. In particular, recombinant molecules of the present invention include transcription control sequences. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the recombinant cells of the present invention. A variety of such transcription control sequences are known to those skilled in the art. Preferred transcription control sequences include those which function in bacterial, yeast, helminths, helminth cells, insect and mammalian cells, such as, but not limited to, *tac*, *lac, trp*, *trc*, oxy-pro, bacteriophage lambda (such as lambda p_{L} and lambda p_{R}), bacteriophage T7, T7*lac*, bacteriophage T3, bacteriophage SP6, bacteriophage SP01, metallothionein, alpha mating factor, *Pichia* alcohol oxidase, alphavirus subgenomic promoters (such as Sindbis virus subgenomic promoters), baculovirus, *Heliothis zea* insect virus, vaccinia virus, adenovirus, simian virus 40, retrovirus actin, Rous sarcoma virus, heat shock, phosphate and nitrate transcription control sequences as well as other sequences capable of controlling gene expression in prokaryotic or eukaryotic cells. Transcription control sequences of the present invention can also include naturally occurring transcription control sequences previously associated with a nucleic acid sequence prior to isolation.

A recombinant molecule of the present invention can be any nucleic acid sequence heretofore described operatively linked to any transcription control sequence capable of effectively regulating expression of the nucleic acid sequence in the cell to be transformed. Preferred recombinant molecules include parasitic helminth nucleic acid sequences capable of hybridizing, under stringent conditions, to at least a portion of *D. immitis* nucleic acid sequence p22L, or to least a portion of *D. immitis* nucleic acid sequence p20.5.
Particularly preferred recombinant molecules contain *D. immitis* nucleic acid sequence p22L, nucleic acid sequences including *D. immitis* p22L, nucleic acid sequence comprising fragments of *D. immitis* p22L, *D. immitis* nucleic acid sequence p20.5, nucleic acid sequences including *D. immitis* p20.5, or nucleic acid sequence comprising fragments of *D. immitis* p20.5. Even more preferred recombinant molecules include pssgal-p4, pHis-p4, pET19b-p4635, ppgal-p22U, pHis-p22U, and pHis-p22U608. Additional even more preferred recombinant molecules include pHis-p39900, p76-80.B3, p105-72.5C, p105-42.1A, pET19b-PLA2417, p76-79-A6, p76-79.C2 and p88-36.1B. The Examples section includes descriptions of the derivations of such recombinant molecules.

Recombinant cells of the present invention include any cells transformed with any nucleic acid sequences of the present invention. Preferred recombinant cells are transformed with recombinant molecules containing at least one of the following: a parasitic helminth nucleic acid sequence capable of hybridizing, under stringent conditions to at least a portion of *D. immitis* nucleic acid sequence p22L, or to least a portion of *D. immitis* nucleic acid sequence p20.5. Particularly preferred recombinant cells are transformed with recombinant molecules including *D. immitis* nucleic acid sequence p22L, nucleic acid sequences including *D. immitis* p22L, nucleic acid sequence comprising fragments of *D. immitis* p22L, *D. immitis* nucleic acid sequence p20.5, nucleic acid sequences including *D. immitis* p20.5, or nucleic acid sequence comprising fragments of *D. immitis* p20.5. Such recombinant cells can also be co-transformed with recombinant molecules including nucleic acid sequences encoding one or more other helminth parasitic proteins including those heretofore disclosed and/or *D. immitis* Di22 and *D. immitis* proteases expressed in L3 and/or L4 larvae, as well as other helminth proteins sharing significant homology with such proteins. The nucleic acid sequence encoding Di22 is disclosed in GenBank data base accession number M82811. The protease is disclosed in Grieve et al., PCT International Publication No. WO 93/10225, published May 27, 1993. Particularly preferred recombinant cells include *E. coli*:pβgal-p4, *E. coli*:pHis-p4, *E. coli*:pET19b-p4₆₃₅, *E. coli*:pβgal-p22U, *E. coli*:pHis-p22U and *E. coli*:pHis-p22U₆₀₈. Additional particularly preferred recombinant cells include *E. coli*:pHis-p39₉₀₀, *S. frugiperda*:p105-72.5C, BHK:p105-42.1A, *E. coli*:pET19b-PLA2₄₁₇, *S. frugiperda*:p76-79-A6p76-79-A6 and BHK:p88-36.1B.

It may be appreciated by one skilled in the art that use of recombinant DNA technologies can improve expression of transformed nucleic acid sequences by manipulating, for example, the number of copies of the nucleic acid sequences within a host cell, the efficiency with which those nucleic acid sequences are transcribed, the efficiency with which the resultant transcripts are translated, and the efficiency of post-translational modifications. Recombinant techniques useful for increasing the expression of nucleic acid sequences of the present invention include, but are not limited to, operatively linking nucleic acid sequences to high-copy number plasmids, integration of the nucleic acid sequences into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of nucleic acid sequences of the present invention to correspond to the codon usage of the host cell, deletion of sequences that destabilize transcripts, and use of control signals that temporally separate recombinant cell growth from recombinant enzyme production during fermentation. The activity of an expressed recombinant protein of the present invention may be improved by fragmenting, modifying, or derivatizing nucleic acid sequences encoding such a protein.

In accordance with the present invention, recombinant cells can be used to produce at least one parasitic helminth protein of the present by culturing such cells under conditions effective to produce such a protein, and recovering the protein. Effective conditions to produce a protein include, but are not limited to, appropriate media, bioreactor, temperature, pH and oxygen conditions that permit protein production. An appropriate medium refers to any medium in which a cell of the present invention, when cultured, is capable of producing parasitic helminth proteins. An effective medium is typically an aqueous medium comprising assimilable carbohydrate, nitrogen and phosphate sources, as well as appropriate salts, minerals, metals and other nutrients, such as vitamins. The medium may comprise complex nutrients or may be a defined minimal medium. Cells of the present invention can be cultured in conventional fermentation bioreactors, which include, but are not limited to, batch, fed-batch, cell recycle, and continuous fermentors. Culturing can also be conducted in shake flasks, test tubes, microtiter dishes, and petri plates. Culturing is carried out at a temperature, pH and oxygen content appropriate for the recombinant cell. Such culturing conditions are well within the expertise of one of ordinary skill in the art. Examples of suitable conditions are included in the Examples section.

Depending on the vector and host system used for production, resultant proteins may either remain within the recombinant cell; be secreted into the fermentation medium; be secreted into a space between two cellular membranes, such as the periplasmic space in *E. coli*; or be retained on the outer surface of a cell or viral membrane. The phrase "recovering the protein" refers simply to collecting the whole fermentation medium containing the protein and need not imply additional steps of separation or purification. Parasitic helminth proteins of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, chromatofocusing and differential solubilization. Isolated parasitic helminth proteins are preferably retrieved in "substantially pure" form. As used herein, "substantially pure" refers to a purity that allows for the effective use of the protein as a therapeutic composition or diagnostic. A vaccine for animals, for example, should exhibit no substantial toxicity and should be capable of stimulating the production of antibodies in a vaccinated animal.

One embodiment of the present invention is the expression of a parasitic helminth protein as a fusion protein which includes the parasitic helminth protein attached to a fusion segment. Such a fusion segment often aids in protein purification, such as permitting one to purify the resultant fusion protein using affinity chromatography. Fusion proteins can be produced by culturing a recombinant cell transformed with a fusion nucleic acid sequence that encodes a protein including the fusion segment attached to either the carboxyl and/or amino terminal end of the parasitic helminth protein. Preferred fusion segments include, but are not limited to, glutathione-S-transferase, β-galactosidase, a poly-histidine segment capable of binding to a divalent metal ion, maltose binding protein and immunoglobulin binding domains (e.g., protein A or portions thereof) with a poly-histidine segment being more preferred. Examples of fusion proteins of the present invention include PβGAL-P4, PHIS-P4, PHIS-P4₆₃₅, PβGAL-P22U, PHIS-P22U, PHIS-P22U₆₀₈, PHIS-P39₉₀₀, and PHIS-PLA2₄₁₇ (also denoted PHIS-P22L₄₁₇).

The present invention also includes antibodies capable of selectively binding to a parasitic helminth protein or mimetope thereof, the protein or mimetope thereof being capable of selectively binding to at least one component of anti-parasitic helminth immune serum. Such antibodies can be either polyclonal or monoclonal antibodies. Antibodies of the present invention include functional equivalents such as antibody fragments and genetically-engineered antibodies, including single chain antibodies, that are capable of selectively binding to at least one of the epitopes of the protein or mimetope used to obtain the antibodies. Preferred antibodies are raised in response to proteins, or mimetopes thereof, that are encoded, at least in part, by a nucleic acid sequence capable of hybridizing, under stringent conditions, to at least a portion of *D. immitis* nucleic acid sequence p22L, or to at least a portion of *D. immitis* nucleic acid sequence p20.5.

A preferred method to produce antibodies of the present invention includes administering to an animal an effective amount of an isolated protein or mimetope thereof to produce the antibody, wherein the protein is capable of selectively binding to at least one component of serum from an animal that is immune to infection by the helminth, the serum being capable of inhibiting helminth development; and recovering the antibodies. Preferably the protein is encoded, at least in part, by a parasitic helminth nucleic acid sequence capable of hybridizing, under stringent conditions, to at least a portion of *D. immitis* nucleic acid sequence p22L, or to at least a portion of *D. immitis* nucleic acid sequence p20.5. Antibodies raised against defined proteins or mimetopes can be advantageous because such antibodies are not substantially contaminated with antibodies against other substances that might otherwise cause interference in a diagnostic assay or side effects if used in a therapeutic composition.

Antibodies of the present invention have a variety of potential uses that are within the scope of the present invention. For example, such antibodies can be used (a) as vaccines to passively immunize an animal in order to protect the animal from parasitic helminth infections, (b) as reagents in assays to detect parasitic helminth infection, and/or (c) as tools to recover desired parasitic helminth proteins from a mixture of proteins and other contaminants.

Furthermore, antibodies of the present invention can be used to target cytotoxic agents to parasitic helminths in order to directly kill helminths expressing proteins selectively bound by the antibodies. Targeting can be accomplished by conjugating (i.e., stably joining) such antibodies to the cytotoxic agents. Suitable cytotoxic agents include, but are not limited to: double-chain toxins (i.e., toxins having A and B chains), such as diphtheria toxin, ricin toxin, *Pseudomonas* exotoxin, modeccin toxin, abrin toxin, and shiga toxin; single-chain toxins, such as pokeweed antiviral protein, α-amanitin, and ribosome inhibiting proteins; and chemical toxins, such as melphalan, methotrexate, nitrogen mustard, doxorubicin and daunomycin. Preferred double-chain toxins are modified to include the toxic domain and translocation domain of the toxin but lack the toxin's'intrinsic cell binding domain.

One embodiment of the present invention is a therapeutic composition capable of protecting an animal from parasitic helminth infection when administered to the animal in an effective manner.

Included in the present invention are therapeutic compositions including at least one of the following: isolated parasitic helminth proteins P22L, and/or P20.5; antibodies that bind selectively to at least one of those proteins; and/or nucleic acid sequences capable of hybridizing, under stringent conditions, to at least a portion of *D. immitis* nucleic acid sequence p22L, and/or p20.5.

Administration of a therapeutic composition containing multiple protective compounds targeting multiple parasitic helminths to an animal can protect the animal from infection by those helminths. Similarly, administration of a therapeutic composition targeting different aspects of a given parasitic helminth may provide additional protection to the animal. For example, a therapeutic composition of the present invention including P4 and/or P22u can also include at least one of the following additional compounds: *D. immitis* P39, *D. immitis* P22L, *D. immitis* P20.5, *D. immitis* Di22, and *D. immitis* proteases expressed in L3 and/or L4 larvae, as well as other helminth proteins sharing significant homology with *D. immitis* P39, *D. immitis* P22L, *D. immitis* P20.5, *D. immitis* Di22, and *D. immitis* proteases expressed in L3 and/or L4 larvae, as well as mimetopes of such proteins, antibodies that selectively bind to such proteins or mimetopes thereof, and nucleic acid sequences encoding such proteins.

As used herein, a protective compound refers to a compound that when administered to an animal in an effective manner is able to treat, ameliorate, and/or prevent infection by a parasitic helminth. Preferred helminths are heretofore disclosed.

Therapeutic compositions of the present invention can be administered to any animal, preferably to mammals, and more preferably to dogs, cats, humans, ferrets, horses, cattle, sheep and other pets and/or economic food animals. Preferred animals to protect include dogs, cats, humans and ferrets, with dogs and cats being particularly preferred.

Therapeutic compositions of the present invention can be formulated in an excipient that the animal to be treated can tolerate. Examples of such excipients include water, saline, Ringer's solution, dextrose solution, Hank's solution, and other aqueous physiologically balanced salt solutions. Nonaqueous vehicles, such as fixed oils, sesame oil, ethyl oleate, or triglycerides may also be used. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer, while examples of preservatives include thimerosal, m or o-cresol, formalin and benzyl alcohol. Standard formulations will either be liquid injectables or solids which can be taken up in a suitable liquid as a suspension or solution for injection. Thus, in a non-liquid formulation, the excipient may comprise dextrose, human serum albumin, preservatives, etc., to which sterile water or saline could be added prior to administration.

In one embodiment of the present invention, the therapeutic composition can also include an immunopotentiator, such as an adjuvant or a carrier. Adjuvants are typically substances that generally enhance the immune response of an animal to a specific antigen. Suitable adjuvants include, but are not limited to, Freund's adjuvant; other bacterial cell wall components; aluminum-based salts; calcium-based salts; silica; polynucleotides; toxoids; serum proteins; viral coat proteins; other bacterial-derived preparations; gamma interferon; block copolymer adjuvants, such as Hunter's Titermax adjuvant (Vaxcel™, Inc. Norcross, GA); Ribi adjuvants (available from Ribi ImmunoChem Research, Inc., Hamilton, MT); and saponins and their derivatives, such as Quil A (available from Superfos Biosector A/S, Denmark). Carriers are typically compounds that increase the half-life of a therapeutic composition in the treated animal. Suitable carriers include, but are not limited to, polymeric controlled release formulations, biodegradable implants, liposomes, bacteria, other viruses, oils, esters, and glycols.

In order to protect an animal from parasitic helminth infection, a therapeutic composition of the present invention is administered to the animal in an effective manner such that the composition is capable of protecting that animal from infection. For example, an isolated protein or mimetope thereof, when administered to an animal in an effective manner, is able to elicit (i.e., stimulate) an immune response, preferably including both a humoral and cellular response, that is sufficient to protect the animal from infection. Similarly, an antibody of the present invention, when administered to an animal in an effective manner, is administered in an amount so as to be present in the animal at a titer that is sufficient to protect the animal from infection, at least temporarily. Nucleic acid sequences of the present invention, preferably oligonucleotides, can also be administered in an effective manner, thereby reducing expression of parasitic helminth proteins in order to interfere with parasite development.

Therapeutic compositions of the present invention can be administered to animals prior to parasite infection in order to prevent infection and/or can be administered to animals after parasite infection in order to treat disease caused by the parasite. For example, proteins, mimetopes thereof, and antibodies thereof can be used as immunotherapeutic agents.

Acceptable protocols to administer therapeutic compositions in an effective manner include individual dose size, number of doses, frequency of dose administration, and mode of administration. Determination of such protocols can be accomplished by those skilled in the art. A suitable single dose is a dose that is capable of protecting an animal from parasitic helminth infection when administered one or more times over a suitable time period. For example, a preferred single dose of a protein, mimetope or antibody therapeutic composition is from about 1 microgram (µg) to about 10 milligrams (mg) of the therapeutic composition for an animal about the size of a dog. Booster vaccinations can be administered from about 2 weeks to several years after the original administration. Preferably booster vaccinations are administered when the immune response of the animal becomes insufficient to protect the animal from parasitic helminth infection. A preferred administration schedule is one in which from about 10 µg to about 1 mg of the vaccine per kg body weight of the animal is administered from about one to about two times over a time period of from about 2 weeks to about 12 months. Modes of administration can include, but are not limited to, subcutaneous, intradermal, intravenous, nasal, oral, transdermal and intramuscular routes.

According to one embodiment, nucleic acid sequences of the present invention can also be administered to an animal in a fashion to enable expression of the nucleic acid sequence into a protective protein in the animal to be protected from parasitic helminth infection. Nucleic acid sequences can be delivered in a variety of methods including, but not limited to, direct injection (e.g., as "naked" DNA or RNA molecules, such as is taught, for example in Wolff et al., 1990, *Science* 247, 1465-1468), packaged as a recombinant virus particle vaccine, and packaged as a recombinant cell vaccine.

A recombinant virus particle vaccine of the present invention includes a recombinant molecule of the present invention that is packaged in a viral coat and that can be expressed in an animal after administration. Preferably, the recombinant molecule is packaging-deficient. A number of recombinant virus particles can be used, including, but not limited to, those based on alphaviruses, pox viruses, adenoviruses, herpes viruses, and retroviruses. Preferred recombinant particle viruses are those based on alphaviruses, with those based on Sindbis virus, Semliki virus, and Ross River virus being more preferred. Methods to produce and use recombinant virus particle vaccines are disclosed in U.S. Patent Application Serial No. 08/015/414, filed February 8, 1993, entitled "Recombinant Virus Particle Vaccines".

When administered to an animal, the recombinant virus particle vaccine infects cells within the immunized animal and directs the production of a parasitic helminth protein or RNA that is capable of protecting the animal from infection by the helminth. For example, when the helminth protein is a *D. immitis* protein, the recombinant virus particle vaccine is administered according to a protocol that results in the animal producing a sufficient immune response to protect itself from heartworm. A preferred single dose of a recombinant virus particle vaccine of the present invention is from about 1 x 10⁴ to about 1 x 10⁵ virus plaque forming units (pfu) per kilogram body weight of the animal. Administration protocols are similar to those described herein for protein-based vaccines.

A recombinant cell vaccine of the present invention includes recombinant cells of the present invention that express at least one parasitic helminth protein. Preferred recombinant cells include *Salmonella, Escherichia coli,* and *Mycobacterium* recombinant cells, with *Salmonella* recombinant cells being more preferred. Such recombinant cells can be administered in a variety of ways but have the advantage that they can be administered orally, preferably at doses ranging from about 10⁸ to about 10¹² bacteria per kilogram body weight. Administration protocols are similar to those described herein for protein-based vaccines. In common with most other enteric pathogens, *Salmonella* strains normally enter the host orally. Once in the intestine, they interact with the mucosal surface, normally to establish an invasive infection. Most *Salmonella* infections are controlled at the epithelial surface, causing the typical *Salmonella*-induced gastroenteritis. Some strains of *Salmonella*, including *S. typhi* and some *S. typhimurium* isolates, have evolved the ability to penetrate deeper into the host, causing a disseminated systemic infection. It appears such strains have the capacity to resist the killing actions of macrophages and other immune cells. *S. typhi* can exist for long periods as a facultative intracellular parasite. Some of the live vaccine strains can also persist for long periods in the mononuclear phagocyte system. Hosts infected in such a manner develop, in addition to a mucosal immune response, systemic cellular and serum antibody responses to the *Salmonella*. Thus, invading *Salmonella*, whether virulent or attenuated, can stimulate strong immune responses, unlike many other enteric pathogens which only set up local, noninvasive gut infections. The potent immunogenicity of live *Salmonella* makes them attractive candidates for carrying parasitic helminth proteins to the immune system.

A preferred recombinant cell-based vaccine is one in which the cell is attenuated. *Salmonella typhimurium* strains, for example, can be attenuated by introducing mutations into genes critical for *in vivo* growth and survival. For example, genes encoding cyclic adenosine monophosphate (cAMP) receptor protein or adenylate cyclase are deleted to produce avirulent, vaccine strains. Such strains can deliver antigens to lymphoid tissue in the gut but demonstrate reduced capacity to invade the spleen and mesenteric lymph nodes. These strains will still stimulate both humoral and cellular immunity in mammalian hosts.

Recombinant cell vaccines can be used to introduce isolated proteins of the present invention into the immune systems of animals. For example, recombinant molecules comprising parasitic helminth nucleic acid sequences of the present invention operatively linked to expression vectors that function in *Salmonella* can be transformed into *Salmonella* host cells. The resultant recombinant cells are then introduced into the animal to be protected. Preferred *Salmonella* host cells are those for which survival depends on their ability to maintain the recombinant molecule (i.e., a balanced-lethal host-vector system). An example of such a preferred host / recombinant molecule combination is a *Salmonella* strain (e.g., UK-1 ᵥ3987 or SR-11 ᵥ4072) which is unable to produce aspartate β-semialdehyde dehydrogenase in combination with a recombinant molecule also capable of encoding the enzyme. Aspartate β-semialdehyde dehydrogenase, encoded by the *asd* gene, is an important enzyme in the pathway to produce diaminopimelic acid (DAP). DAP is an essential component of the peptidoglycan of the cell wall of Gram-negative bacteria, such as *Salmonella*, and, as such, is necessary for survival of the cell. Thus, *Salmonella* lacking a functional *asd* gene can only survive if they maintain a recombinant molecule that is also capable of expressing a functional *asd* gene.

The efficacy of a therapeutic composition of the present invention to protect an animal from infection by a parasitic helminth can be tested in a variety of ways including, but not limited to, detection of protective antibodies (using, for example, proteins or mimetopes of the present invention), detection of cellular immunity within the treated animal, or challenge of the treated animal with the parasitic helminth or antigens thereof to determine whether the treated animal is resistant to infection. Such techniques are known to those skilled in the art.

One preferred embodiment of the present invention is the use of *D. immitis* nucleic acids and proteins to protect an animal from heartworm infection. It is particularly preferred to prevent L3 larvae that are delivered to the animal by the mosquito intermediate host from maturing into adult worms. As such, preferred therapeutic compositions are those that are able to inhibit at least one step in the portion of the parasite's development cycle that includes L3 larvae, third molt, L4 larvae, fourth molt, immature adult prior to entering the circulatory system. In dogs, this portion of the development cycle is about 70 days.

In another embodiment of the present invention, preferred nucleic acid sequences, proteins, and antibodies to protect an animal against heartworm include *D. immitis* p22L, and/or *D. immitis* p20.5, as well as nucleic acid sequences including at least a portion of *D. immitis* p22L and/or *D. immitis* p20.5, proteins encoded by those sequences, mimetopes of such proteins, and antibodies that selectively bind to such proteins. Particularly preferred therapeutic compositions include proteins that share at least some *D. immitis* P22L/P20.5 epitopes.Such compositions are administered to animals in a manner effective to protect the animals from heartworm infection. Additional protection may be obtained by administering additional protective compounds, including other *D. immitis* antigens, such as *D. immitis* P4, *D. immitis* P22U, *D. immitis* Di22, and/or *D. immitis* proteases expressed in L3 and/or L4 larvae.

It is also within the scope of the present invention to use the isolated parasitic helminth proteins, mimetopes, nucleic acid sequences, and antibodies as diagnostic agents. Preferably such diagnostic agents are supplemented with additional compounds that can detect other phases of the helminth's life cycle.

One embodiment of the present invention is a therapeutic composition capable of protecting an animal from parasitic helminth infection when administered to the animal in an effective manner that includes a compound capable of substantially interfering with the function of a parasitic helminth protein LDLr class A cysteine-rich motif, preferably by reducing the ability of such a protein to take up sterols. As used herein, a parasitic helminth protein LDLr class A cysteine-rich motif, or LDLr class A motif, refers to cysteine-rich motifs in parasitic helminth proteins that are homologous to that identified in *D. immitis* P4. Such motifs also occur in several other proteins, including LDL receptor-related proteins and α₂-macroglobulin receptors, as heretofore disclosed. As used herein, substantially interferes refers to the ability of the compound to inhibit parasitic helminth development.

Preferred therapeutic compositions are those that are targeted to the LDLr class A motif shared by *D. immitis* P4 and other parasitic helminth proteins encoded, at least in part, by a nucleic acid sequence capable of hybridizing, under stringent conditions, to at least a portion of *D. immitis* p4. Suitable compounds can be identified by a variety of methods, including known methods to screen inorganic and organic molecules and rational drug design methods in which the active site of the motif is identified and a compound designed that would interfere with that active site. Suitable compounds are likely to include sterol mimetopes that are capable of interfering with sterol uptake by parasitic helminths, possibly by selectively binding to the LDLr class A motif.

Parasitic helminths, some protozoans and some insects are not able to synthesize squalenes and sterols *de novo*. Thus, parasitic helminths require sterols as precursors for steroid hormones and as integral structural components of cellular membranes. Cholesterol, one of the sterols that parasitic helminths cannot produce *de novo*, regulates cellular function, growth and differentiation by interacting with a number of protein kinases, protein receptors and ion pumps. Cholesterol is also the precursor of ecdysteroids, the steroidal molting hormones of insects, also believed to serve a similar function in parasitic helminths. While not being bound by theory, it is believed that the LDLr class A motif is important in the development of parasitic helminths (including nematodes, trematodes, and cestodes) as well as other organisms that do not synthesize sterols *de novo* (e.g., some parasitic protozoans and insects), because known LDLr class A motifs are apparently involved in sterol uptake. Such motifs in LDL receptors, for example, are responsible for binding the positively-charged ligands apolipoprotein B (apo B) and apolipoprotein E (apo E) within lipoprotein particles (see, for example, Herz et al., 1988, *EMBO J.* 7, p. 4119-4127). Apo E is involved in the clearance of triglyceride-rich lipoproteins and in reverse cholesterol transport. ApoE is also thought to be involved in the modulation of cell growth in mammalian lymphocytes as well as in brain and other tissues. Thus, compounds having the ability to interfere with sterol uptake by parasitic helminths due to their ability to interact with LDLr class A motifs are attractive as therapeutic compositions of the present invention.

Such therapeutic compositions can be administered to animals in an effective manner to protect animals from parasitic helminth infection. Effective amounts and dosing regimens can be determined using techniques known to those skilled in the art.

The following examples are provided for the purposes of illustration and are not intended to limit the scope of the invention.

### Examples

### Example 1

This Example describes a procedure for producing and evaluating immune sera of the present invention.

Four dogs were immunized with chemically-abbreviated *D. immitis* larval infections (using the method described in Grieve et al., 1988, *ibid*.), and two dogs served as chemically-treated controls. The dogs were housed in indoor mosquito-free individual cages at a temperature of about 22°c and about 40% to about 65% humidity. On day 532, post initial immunization, each dog was challenged with about 100 L3 *D. immitis* larvae by implanting 5 diffusion chambers per dog, each diffusion chamber containing about 20 L3 *D. immitis* larvae, using the method described in Grieve et al., 1988, *ibid.* Concomitant with chamber implantation, each dog was injected subcutaneously with about 50 L3 *D. immitis* larvae, and the infection was allowed to proceed beyond the anticipated prepatent period. Challenge infections were repeated on day 588, post initial immunization, both by implanting 5 diffusion chambers per dog, each chamber having about 20 L3 *D. immitis* larvae and by subcutaneously inoculating about 30 L3 *D. immitis* larvae per dog. Serum samples were collected from the immunized dogs at numerous time points throughout the study period. Serum samples were analyzed for antibodies that selectively bound to L3 and/or L4 surface antigens using an indirect fluorescent antibody assay, and for antibodies that selectively bound to L3 soluble antigens, L4 soluble antigens and/or to an excretory/secretory antigen fraction using an indirect ELISA, as described by Grieve et al., 1988, *ibid.* The results indicated that serum from dogs that had been immunized and challenged with *D. immitis* larvae had produced antibodies to both surface and soluble *D. immitis* larval antigens. The sera were pooled, and those obtained from larval-immunized dogs (i.e., anti-larval immune sera) were shown to inhibit larval development; see, for example, Example 2. Immune sera were also shown to selectively bind to L3 and/or L4 larval proteins having molecular weights of about 15 kD, 23/24 kD doublet, 31 kD, 33 kD, 39 kD, 42 kD, 55 kD, 59 kD, 66 kD, 70 kD, 97 kD and 207 kD by Tris-glycine SDS PAGE.

### Example 2

This Example demonstrates that serum collected from larval-immunized dogs, produced as described in Example 1, is capable of inhibiting parasite development whereas serum collected from non-immunized dogs is not.

One subcutaneous pocket was formed in each of about 3 to about 6 Balb/C BYJ mice that were about 10 weeks old. One diffusion chamber, containing 20 L3 *D. immitis* larvae, was implanted into each pocket alone with 0.5 ml of sera collected from immunized dogs or from non-immunized dogs, produced as described in Example 1. The diffusion chambers were recovered two or three weeks later. Living larvae in the chambers were counted and placed into glacial acetic acid, followed by 70% ethanol containing 5% glycerin. The ethanol was allowed to evaporate leaving the larvae in glycerin. The larvae were measured using projected images in the Macmeasure image analysis system on a Macintosh computer.

Three experiments, in which different serum samples were exposed to larvae in diffusion chambers, were conducted: Experiment 1 compared equal portions of sera collected from individual dogs at days 56, 77 and 117 after challenge. Experiments 2 and 3 compared serum collected from immunized dogs 117 days after initial challenge to control sera. In experiment 2, the control serum was a pool of sera collected from 12 naive dogs; in experiment 3, control serum was collected from a single naive dog. Each of the experiments also included controls in which the larvae were not exposed to any serum.

In experiment 1, chambers were recovered two weeks post-inoculation. The number of larvae retrieved from chambers implanted in mice receiving serum from immunized (i.e., immune) dogs was lower than that of larvae in chambers implanted in mice receiving naive dog serum, but the difference was not statistically significant. Also, no differences were seen between the length of larvae regardless of which serum was used.

In experiments 2 and 3, the chambers were recovered three weeks after infection. There were significant differences in the larval recoveries between those receiving serum from naive dogs and those from immune dogs; there were about 34% more larvae recovered from mice treated with naive dog serum than were recovered from mice treated with immune serum. The lengths of the larvae were also significantly shorter in those chambers exposed to sera from immune dogs compared to larvae in chambers exposed to naive dog sera. Thus, this Example shows that serum collected from dogs immune to *D. immitis* infection inhibits larval development, compared to serum collected from naive dogs.

### Example 3

This Example describes the purification of *D. immitis* P22U, P22L and P20.5 as well as tryptic digestion of the proteins, and partial amino acid sequencing of several tryptic fragments.

Larvae and ES proteins were pulse chase labeled as described by Frank et al. , 1992, *J. Parasitol.* 77, 950-956. Proteins of 22 and 20.5 kD were shown to be developmentally regulated proteins that were particularly present in L3 and L4.

Third stage larvae were collected and cultured *in vitro* as described in Frank et al., *ibid.* The larvae were washed free of serum proteins at about 48 hr, placed back into culture and the serum-free media containing larval ES products was collected from 48 to 144 hr in culture. Each week's yield of ES was collected, filtered through a 0.45 µm filter (Acrodisc™, Gelman Sciences, Ann Arbor, Michigan) and frozen at about -70°C until further processing. Processing was conducted at about 4°C or on ice and consisted of thawing the ES and adding 0.5 M EDTA·Na₂, pH 8.0, to a final concentration of 5 mM. EDTA was the only protease inhibitor used since only metalloprotease activity has been found in larval ES (Richer et al., 1992, *Exp. Parasit*. 75, p. 213-222). The ES was concentrated and the buffer was exchanged using Centriprep-10 and Centricon-10 (Amicon, Beverly, MA); the final buffer was 20 mM Tris, 1 mM EDTA·Na₂, pH 7.2.

All chromatography was performed on a Beckman 338 binary system using System Gold version 3.10 chromatography software (Beckman Instruments, Inc., San Ramon, CA). The separations and fraction collections were conducted at room temperature and the fractions placed at about 4°C immediately after each run. When portions of the samples were metabolically labeled, aliquots of the collected fractions were assayed in scintillation fluid by a Beckman Model LS 1801 liquid scintillation counter (Beckman Instruments, Inc.).

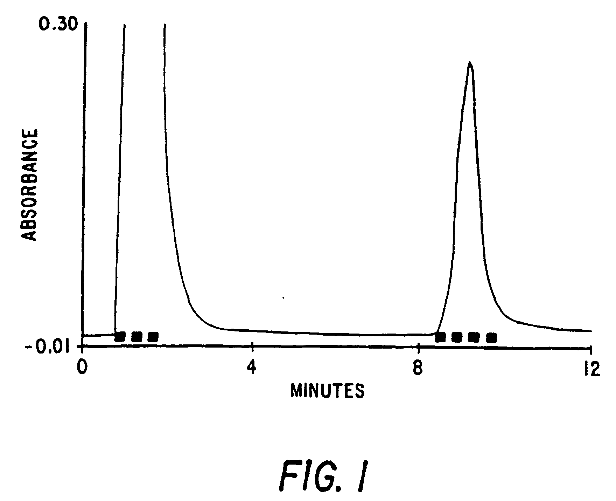

The first purification was from approximately 38,650 larvae, 3,550 of which had been metabolically labeled with Translabel™ from about 48 to 144 hr. The ES products were concentrated to 175 µl in 20 mM Tris, 1 mM EDTA·Na₂, pH 7.2 (Buffer A) and contained 1.3 µg/µl protein with an ³⁵S-incorporation of 7,450 cpm/µl. Cation exchange chromatography was used as the first step in purification. A SynChropak CM300-GRD 4.6 x 50 mm column (Synchrom, Inc., Lafayette, Indiana) was used. The sample was diluted with 300 µl buffer A, centrifuged at 12,000 g and the supernate injected onto the column at 0.5 ml/min Buffer A. After a 5 min wash, the adsorbed proteins were eluted with a steep gradient to 100% Buffer B (1 M KCl in Buffer A) over 0.1 min while 200 µl fractions were collected throughout. Detection of proteins was at 280 nm. FIG. 1 shows the resultant chromatogram. Boxed fractions, designated 4, 5, 6, 23, 24, 25 and 26, were evaluated by SDS PAGE.

The vast majority of contaminating proteins eluted in the initial peak. In contrast, P22U, as well as P22L and P20.5, eluted in the second peak, i.e., in fractions 23, 24, 25 and 26.

Reverse phase chromatography using a Vydac C₄ 0.21 x 25 cm, 5 µm particle size column (Vydac 214TP52, The Separations Group, Hesperia, CA) was used to separate P22U from P22L and P20.5. Buffer C consisted of 0.1% trifluoroacetic acid (TFA), 0.085% triethylamine (TEA) in Milli-Q water produced by processing 18 megaohm water through a Milli-Q Plus water system (Millipore Corp., Bedford, MA), while Buffer D consisted of 0.085% TFA, 0.085% TEA, 80% CH₃CN in Milli-Q water. Detection of proteins was at 220 nm. Fractions 23 and 24 from the cation exchange run were injected onto the column followed by fractions 25 and 26 two min later. The initial flow rate was 0.25 ml/min at 12.5% D, 87.5% C. The flow rate was reduced to 0.17 ml/min at 4 min and a gradient to 62.5% D over 200 min was started at 6 min. Fractions of 0.75 min were collected.

Aliquots of peak fractions were submitted to SDS-PAGE and analyzed by silver staining and autoradiography. P20.5 appeared first and predominated in fractions 99-102 (elution times of from about 74.25 minutes through about 76.5 minutes). P22L predominated in fractions 103-107 (elution times of from about 77.25 minutes through about 80.25 minutes), although there was significant contamination with P20.5. P22U eluted much later, in fractions 229-235 (elution times of from about 171.75 minutes through about 176.25 minutes). P22U, P22L and P20.5 recovered from C4 reverse phase chromatography were each shown by immunoblot analysis (see, for example Grieve et al., 1992, *J. Immunol.* 148, 2511-2515 for method), using dog immune serum prepared as in Example 1, to be uniquely recognized by the immune serum.

The molecular weights of P22U, P22L and P20.5 were determined using Tris-tricine SDS-PAGE according to the method of Schagger and von Jagow, 1987, *Analyt. Biochem.* 166, 368-379. This Tris-tricine system has been reported to give more accurate estimates of molecular weights for other proteins; see, for example, Patton et al., 1991, *Analyt. Biochem.* 197, 25-33. Molecular weight standards used were SDS-PAGE Standards, Low Range (Bio-Rad Laboratories) and MW-SDS-17S (Sigma Chemical Co., St. Louis, MO). The 20 and 22L kD proteins resolved as 16.1 and 18.8 kD by reducing Tris-tricine SDS-PAGE. This same sample electrophoresed on 1) a second Tris-tricine gel resulted in molecular weights of 15.3 and 17.7 kD, and 2) a Tris-glycine gel resulted in molecular weights of 21.9 and 23.2 kD.

Fractions containing P22L and P20.5 obtained from C₄ reverse phase chromatography were submitted to C₁₈ reverse phase chromatography using a 0.21 x 25 cm, 5 µm particle size column (Vydac 218TP52) to try to separate the two proteins further. The flow rate was 0.2 ml/min at 11.1% Buffer F (0.085% TFA, 90% CH₃CN in Milli-Q water), 88.9% Buffer E (0.1% TFA in Milli-Q water) with a gradient to 83.3% Buffer F over 65 min. One minute fractions were collected from 3 through 83 min. P20.5 eluted first, followed by P22L.

A sample of purified P22U obtained from C₄ reverse phase chromatography as well as samples of purified P22L and P20.5 obtained from C₁₈ reverse phase chromatography were denatured, reduced and pyridylethylated by standard procedures (see, for example, Matsudaira, P. T. (ed.)., 1989, *A Practical Guide to Protein and Peptide Purification for Microsequencing*). The pyridylethylated P22U, P22L and P20.5 samples were each subjected to trypsin digestion, and the tryptic peptides separated by C₁₈ reverse phase chromatography using a 0.21-cm x 25-cm, 5-µm particle size column (Vydac 218TP52) by a procedure based on Stone et al., 1989, in Matsudaira, P. T. (ed.)., *A Practical Guide to Protein and Peptide Purification for Microsequencing,* p. 31-47.

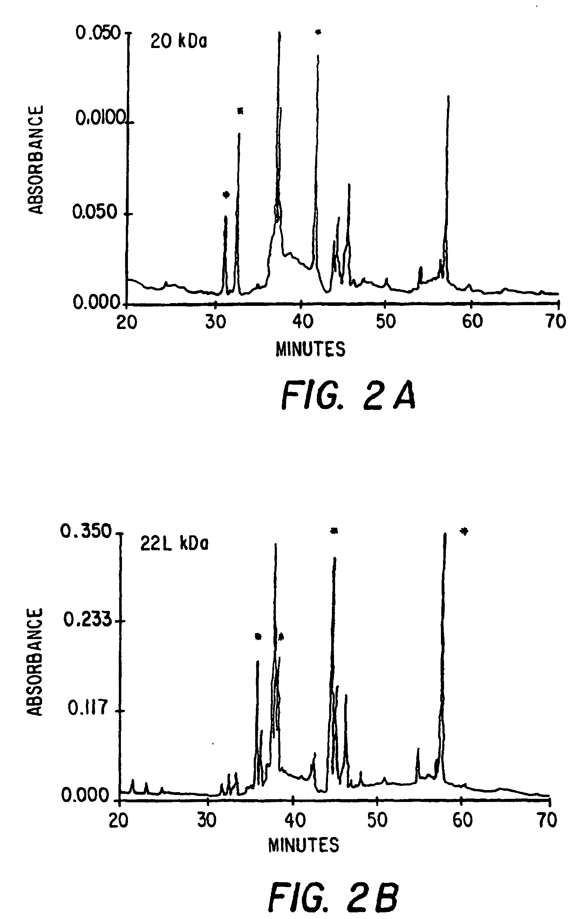

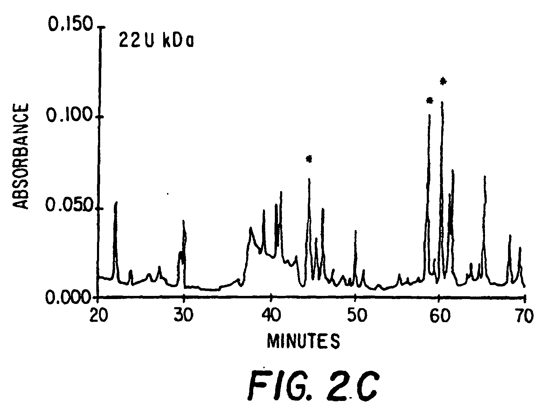

Chromatograms depicting the tryptic fragments of P20.5, P22L and P22U are shown in FIG. 2, labelled, respectively as "20 kDa", "22L kDa" and "22U kDa". As seen from FIG. 2, the tryptic map of P22U is completely different from those of P22L and P20.5, which share at least some fragments in common.

Fragments indicated by asterisks were submitted for sequencing. All sequencing was conducted at Macromolecular Resources, Department of Biochemistry, Colorado State University, Fort Collins, CO. The peptides were concentrated to 50 µl or less using a Speedvac® and frozen at about -20°C until sequencing. N-terminal sequencing was conducted in an ABI Model 473A Protein/Peptide Sequencer System (Applied Biosystems, Inc., Foster City, CA) using pulsed liquid chemistry and on line microgradient PTH amino acid analysis (see, for example, Hewick et. al., 1981, *J. Biol. Chem.* 256, p. 7990-7997; Geisow and Aitken, 1989, in Findlay, J.B.C. and M.J. Geisow (ed.). *Protein Sequencing: A Practical Approach*, p. 85-98).

N-terminal amino acid sequencing of electroblotted P22L and P20.5 was conducted as originally described by Matsudaira, 1987, *J. Biol. Chem.* 262, 10035-10038, and outlined by LeGendre et al., 1989, "A Practical Guide to Protein and Peptide Purification for Microsequencing," Matsudaira, P.T. (ed.), pp. 49-69. P20.5 yielded sequence while P22L was determined to be N-terminally blocked.

The most likely sequence of the P22U tryptic fragment eluting at 44 minutes (referred to as the 44 min P22U tryptic fragment), using one-letter amino acid code, was MAQDAFPNACAQGEPK. The most likely sequence of the P22U tryptic fragment eluting at 58 minutes (referred to as the 58 min P22U tryptic fragment) was AIAPCQLTAVQSVLPCADQCQK. The most likely sequence of the P22U tryptic fragment eluting at 60 minutes (referred to as the 60 min P22U tryptic fragment) was LGSCSPDCGLDLPSDNVMVQDV.

The most likely sequence of the P22L tryptic fragment eluting at 35 minutes (referred to as the 35 min P22L tryptic fragment) was HVETHEACYDQR. The most likely sequence of the P22L tryptic fragment eluting at 38 minutes (referred to as the 38 min P22L tryptic fragment) was GEFVESDGK. The most likely sequence of the P22L tryptic fragment eluting at 44 minutes (referred to as the 44 min P22L tryptic fragment) was N-WQCSYD. The most likely sequence of the P22L tryptic fragment eluting at 58 minutes (referred to as the 58 min P22L tryptic fragment) was EPQSWCILKPHQS-TQR.

The most likely sequence of the N-terminus of P20.5 was ETQEETV-FEE-D-D. The most likely sequence of the P20.5 tryptic fragment eluting at 31 minutes (referred to as the 31 min P20.5 tryptic fragment) was FVESDGK. The most likely sequence of the P20.5 tryptic fragment eluting at 32 minutes (referred to as the 32 min P20.5 tryptic fragment) was T-EACYDQR. The most likely sequence of the P20.5 tryptic fragment eluting at 42 minutes (referred to as the 42 min P20.5 tryptic fragment) was FNWQCSYD.

### Comparative Example 4

This Example describes the cloning and sequencing of *D. immitis* nucleic acid sequence p4. *D. immitis* p4 was identified by its ability to encode a protein that selectively bound to at least one component of immune serum collected from a dog immunized with *D. immitis* larvae.

*D. immitis* L3 larvae were harvested from mosquitos using standard techniques and cultivated *in vitro* in 50:50 NCTC-135/IMDM (NI) media (Sigma) supplemented with 20% serum supplement at 37°C, 5% carbon dioxide for 48 hours. Total RNA was extracted from the larvae using an acid-guanidinium-phenol-chloroform method similar to that described by Chomczynski and Sacchi, 1987, *Anal. Biochem.* 162, p. 156-159. Approximately 15,000 to 30,000 larvae were used in an RNA preparation. Poly A+ selected RNA was separated from total RNA by oligo-dT cellulose chromatography using Oligo dT cellulose from Collaborative Research, Inc., Waltham, MA, according to the method recommended by the manufacturer.

A *D. immitis* L3 larval cDNA expression library was constructed in lambda (λ) Uni-ZAP" XR vector (available from Stratagene Cloning Systems, La Jolla, CA) using Stratagene's ZAP-cDNA Synthesis Kit® protocol and about 5 µg to about 6 µg of L3 poly A+. The resultant library was amplified to a titer of about 4.88 x 10⁹ pfu/ml with about 97% recombinants.

Using the protocol described in the Stratagene picoBlue immunoscreening kit, the L3 larval cDNA expression library was screened with immune dog serum prepared as described in Example 1. Antibodies specific for a highly immunoreactive protein termed the "ladder protein" (Culpepper et al., 1992, *Mol. Biochem. Parasitol.* 54, p. 51-62) had been adsorbed from this serum by affinity chromatography with a recombinant GST-ladder fusion protein. Immunoscreening of duplicate plaque lifts of the cDNA library with the same serum identified 4 positive clones, one of which included *D. immitis* nucleic acid sequence p4. The remaining 3 clones were shown to encode at least portions of P39 and are described in greater detail in Example 8.

The plaque-purified clone including *D. immitis* nucleic acid sequence p4 was converted into a double stranded recombinant molecule, herein denoted as pβgal-p4, using R408 helper phage and XL1-Blue *E. coli* according to the *in vivo* excision protocol described in the stratagene ZAP-cDNA Synthesis Kit. Double stranded plasmid DNA was prepared using an alkaline lysis protocol, such as that described in Sambrook et al., *ibid.* The plasmid DNA was digested with *Eco*RI and *Xho*I restriction endonucleases to release two *D. immitis* DNA fragments of about 580 and 320 nucleotides, the entire *D. immitis* p4 fragment being about 900 nucleotides in size.

A p4 nucleic acid probe detected specific *D. immitis* genomic DNA restriction fragments on a Southern blot verifying the *D. immitis* origin of the p4 cDNA clone.

The plasmid containing *D. immitis* p4 was sequenced using the Sanger dideoxy chain termination method, as described in Sambrook et al., *ibid.* The Promega Erase a Base method (available from Promega Corp., Madison, WI) was used to generate deletion clones for sequence analysis . An about 913-nucleotide consensus sequence of the entire *D. immitis* p4 DNA fragment was determined and is presented as SEQ ID NO:1. The entire 913 nucleotides form an open reading frame encoding an amino acid sequence of about 303 amino acids, presented in SEQ ID No:2. The first ATG codon within this sequence spans nucleotides from about 417 through about 419. As such, SEQ ID NO:1 does not encode a full-length protein, but does encode a protein that selectively binds to at least one component of immune dog serum. The predicted size of the protein encoded by SEQ ID NO:1 is about 35.5 kD, with an estimated pI of about 4.26.

A homology search of the non-redundant protein sequence database was performed through the National Center for Biotechnology Information using the BLAST network. This database includes SwissProt + PIR + SPUpdate + GenPept + GPUpdate. The search was performed using SEQ ID No:2 and showed the only significant homology shared between SEQ ID. NO:2 and known sequences to be a contiguous stretch of 9 amino acids, namely DDCGDGSDE, that was also found in human LDL-receptor related protein, human and mouse alpha-2-macroglobulin receptors and rat renal GP 330 glycoprotein. A conserved stretch of eight of the nine amino acids is also found in *Caenorhabditis elegans* LDL receptor-related protein and *C. elegans* basement membrane proteoglycan.

### Comparative Example 5

This Example demonstrates the ability of *D. immitis* p4 to encode a protein that selectively binds to immune serum. The recombinant protein is also capable of generating an immune response in rabbits.

Recombinant molecule pET19b-p4₆₃₅, containing *D. immitis* p4 nucleotides from about 1 through about 635 operatively linked to bacteriophage T7*lac* transcription control sequences and to a fusion sequence encoding a poly-histidine segment comprising 10 histidines was produced in the following manner. An about 635-nucleotide DNA fragment containing nucleotides spanning from about 1 through about 635 of SEQ ID NO:1, called p4₆₃₅, was PCR amplified from a clone containing *D. immitis* p4 using the primers 5' CGGGATCCCG AGTTAAATAG TCG 3' (denoted 394-5': *Bam*HI site underlined) and 5' TGCAGGATCC TGCACCG 3' (denoted 394-3'; *Bam*HI site underlined). The PCR product was digested with *Bam*HI restriction endonuclease, gel purified and subcloned into expression vector pET19b (available from Novagen Inc., Madison, WI) that had been cleaved with *Bam*HI. The resulting recombinant molecule pET19b-p4₆₃₅ was transformed into *E. coli* BL21(DE3)pLysS to form recombinant cell *E. coli*:pET19b-p4₆₃₅. *E. coli* BL21(DE3)pLysS includes a bacteriophage T7 RNA polymerase gene under the control of *lac* transcription control sequences.

Recombinant cell *E. coli*:pET19b-p4₆₃₅ was cultured in shake flasks containing an enriched bacterial growth medium containing 0.1 mg/ml ampicillin and 0.034 mg/ml chloramphenicol at about 37°C. When the cells reached an optical density at about 600 nanometers (OD₆₀₀) of about 0.819, expression of *D. immitis* p4 was induced by addition of about 1mM isopropyl-β-D-thiogalactoside (IPTG). Protein production was monitored by SDS PAGE of recombinant cell lysates, followed by Coomassie blue staining, using standard techniques. Recombinant cell *E. coli*:pET19b-p4₆₃₅ produced a protein, denoted PHIS-P4₆₃₅, that migrated with an apparent molecular weight of about 37 kD. Such a protein was not produced by cells transformed with the pET-19b plasmid lacking a *D. immitis* DNA insert.

Immunoblot analysis of recombinant cell *E. coli*:pET19b-p4₆₃₅ lysates indicates that the 37 kD protein is able to selectively bind to immune dog serum and, as such, is capable of binding to at least one component of a serum that is capable of inhibiting *D. immitis* larval development.

The *E. coli*:pET19b-p4₆₃₅ histidine fusion peptide was separated from soluble *E. coli* proteins by nickel chelation chromatography and an imidazole gradient. Immunoblot analysis of the total *E. coli*:pET19b-p4₆₃₅ lysate, column eluate and column void volume indicates that the 37kD protein can be isolated on the nickel column and is able to selectively bind to immune dog serum, and as such, is capable of binding to at least one component of a serum that is capable of inhibiting *D. immitis* larval development. The column eluate was not detected by preimmune sera from the same immune dog.

A rabbit was immunized with recombinant PHIS-P4₆₃₅ according to standard techniques, such as those heretofore disclosed. Antibodies collected from the immunized rabbit were capable of binding to formic acid-cleaved PHIS-P4₆₃₅, indicating that the antibodies were capable of detected unfused P4₆₃₅.

### Comparative Example 6

This Example describes the isolation and sequence of *D. immitis* nucleic acid sequence p22U.

Total RNA was extracted from adult female *D. immitis* worms, poly A+ RNA prepared, and an adult female *D. immitis* cDNA library produced, using methods similar to those described in Example 4.

A segment of DNA for use in the identification of a nucleic acid sequence capable of encoding at least a portion of P22U was produced by PCR amplification using standard techniques, such as those described in Sambrook et al. , *ibid.* Briefly, first strand cDNA was synthesized from adult female poly A+ RNA using Murine Leukemia Virus reverse transcriptase (available from Stratagene) and Stratagene's linker-primer from their ZAP-cDNA Synthesis Kit, namely 5' GAGAGAGAGA GAGAGAGAGA ACTAGTCTCG AGTTTTTTTT TTTTTTTTTT 3' . A pool of two sets of degenerate primers was produced based on the partial amino acid sequence of the 60 min tryptic fragment described in Example 3. One degenerate set of primers, denoted GRF 11, includes the following sequences: 5'TGY TCN CCN GAY TGY GG 3', wherein Y can be either C or T, and N can be either A, G, C or T. The second set of primers, denoted GRF 12, includes the following sequences: 5'TGY AGT CCN GAY TGY GG 3'. PCR amplification using the pool of degenerate primers in combination with Stratagene's linker-primer as the antisense primer was used to amplify the DNA segment. Verification that the appropriate segment had been amplified was accomplished by Southern blot analysis using a degenerate probe based on a more C-terminal amino acid sequence of the 60 min tryptic fragment, namely GRF 3 which includes the following sequences: 5' TGN ACC ATN ACR TTR TC 3', wherein R can be either A or G.

The amplified segment was gel purified, electroeluted and cloned into the pCR II cloning vector (available from Invitrogen, San Diego, CA), following the manufacturers' instructions. Two clones were partially sequenced, yielding a nucleic acid sequence which included a sequence corresponding to the amino acid sequence of the 60 min tryptic fragment. The nucleic acid sequence includes from nucleotides about 444 to about 696 of SEQ ID NO:3, described in more detail below.

The adult female cDNA library was screened with an antisense probe, using stringent (i.e., standard) hybridization conditions as described in Sambrook et al., *ibid*.. The antisense probe, denoted GRF14, was based on the DNA sequence derived from the amplified segment and has the sequence 5' CTGTTTGAAC CATAACATTA TCAGATGG 3'. Plaques which hybridized to the probe were rescreened, plaque purified and clones containing *D. immitis* nucleic acid sequence p22U (i.e., clones that hybridized with the antisense probe and having the apparent nucleic acid sequence designated in SEQ ID NO:3) were submitted to nucleic acid sequencing as described in Example 4.

An about 1016-nucleotide consensus sequence of *D. immitis* nucleic acid sequence p22U was determined and is presented as SEQ ID NO:3. The deduced translation product is presented both with SEQ ID NO:3 and in SEQ ID NO:4. SEQ ID NO:3 apparently encodes a protein of about 208 amino acids, the sequence including a stop codon spanning nucleotides about 627 through about 629. There are two ATG codons spanning nucleotides about 39 to about 41 and spanning nucleotides about 57 to about 59. Although SEQ ID NO:3 encodes a protein of about the expected size (i.e., predicted size of about 22 kD), the actual translation initiation site of the protein is as yet unknown.

Nucleic acid sequences encoding all three partially sequenced tryptic peptides are included in SEQ ID NO:3, indicating that the sequence does encode at least a portion of P22U. The portion of the 44 min tryptic fragment that was sequenced spans amino acids about 77 to about 92 of SEQ ID NO:4 and agrees with the derived sequence in all but one amino acid. The portion of the 58 min tryptic fragment that was sequenced spans amino acids about 27 to about 48 of SEQ ID NO:4 and agrees with the derived sequence in all but one amino acid. The portion of the 60 min tryptic fragment that was sequenced spans amino acids about 145 to about 166 of SEQ ID NO:4 and agrees with the derived sequence in all but one amino acid. A homology search of the non-redundant protein sequence database was performed through the National Center for Biotechnology Information using the BLAST network. This database includes SwissProt + PIR + SPUpdate + GenPept + GPUpdate. The search was performed using SEQ ID NO:4 and no significant homology with known proteins was indicated.

### Comparative Example 7

This Example demonstrates the ability of *D. immitis* p22U to encode a protein that selectively binds to immune serum and to antibody against native p22U.

Recombinant molecule pHis-p22U₆₀₈, containing *D. immitis* p22U nucleotides from about 41 through about 649 operatively linked to trc transcription control sequences and to a fusion sequence encoding a poly-histidine segment comprising 6 histidines was produced in the following manner. An about 608-nucleotide DNA fragment containing nucleotides spanning from about 41 through about 649 of SEQ ID NO:3, called p22U₆₀₈, was PCR amplified from a clone containing *D. immitis* p22U using the primers 5' GTTGCAATAT GGGATCCAAT GAGCC 3' (denoted 22USEN; *Bam*HI site underlined) and 5' CGCTAGTGCA GGATCCTCAA TACTC 3' (denoted 22UANT; *Bam*HI site underlined). The PCR product was digested with *Bam*HI restriction endonuclease, gel purified and subcloned into expression vector pTrcHisB (available from Invitrogen) that had been cleaved with *Bam*HI. The resulting recombinant molecule pHis-p22U₆₀₈ was transformed into *E. coli* to form recombinant cell *E. coli*:pHis-p22U₆₀₈. The recombinant cell was cultured in shake flasks containing an enriched bacterial growth medium containing 0.1 mg/ml ampicillin at about 37°C. When the cells reached an OD₆₀₀ of about 0.3, expression of *D. immitis* p22U₆₀₈ was induced by addition of about 1 mM IPTG. Protein production was monitored by SDS PAGE of recombinant cell lysates, followed by Coomassie blue staining, using standard techniques. Recombinant cell *E. coli*:pHis-p22U₆₀₈ produced a protein, denoted herein as PHIS-P22U₆₀₈, that migrated with an apparent molecular weight of about 27 kD. Such a protein was not produced by cells transformed with the pTrcHisB plasmid lacking a *D. immitis* DNA insert.

Immunoblot analysis of recombinant cell *E. coli*:pHis-p22U₆₀₈ lysates indicates that the 27-kD protein is able to bind to immune dog serum and, as such, is capable of binding to at least one component of a serum that is capable of inhibiting *D. immitis* larval development. Immune dog serum essentially does not bind to lysates of cells transformed with only the pTrcHisB plasmid. Cats have been immunized with PHIS-P22U₆₀₈ according to standard protocols.

Native *D. immitis* P22U was purified and used to immunize a rabbit for reagent antibody. Antisera collected from the immunized rabbit detected both native 22U protein from 6 day L4 larvae and PHIS-P22U₆₀₈ recombinant protein by Western blot.

### Comparative Example 8

This Example describes the cloning and sequence of *D. immitis* nucleic acid sequence p39. *D. immitis* p39 was identified by its ability to encode a protein that selectively bound to at least one component of immune serum collected from a dog immunized with *D. immitis* larvae.

Genomic and cDNA expression libraries in λZapII (Short, J.M., et al., 1988, *Nucleic Acids Res* 16:7583-7600), a derivative of λgtll, were prepared from total genomic DNA, or L4 or L3 larval stage mRNAs, respectively, using standard procedures (*Short Protocols in Molecular Biology,* 1989, Ausubel, M.F., et al., eds.). In this example, four cDNA expression libraries were prepared from *D. immitis* adult female, adult male, 48 hour third stage (L3), and 6 day fourth stage (L4) larvae mRNA in lambda (λ) Unizap XR vector (Stratagene) using the Zap-cDNA synthesis kit® protocol (Stratagene) in a manner similar to that described in Example 4. For each library approximately 5-6 µg of mRNA was used. The resulting libraries were amplified one time and the titers were:
Adult male: 1.15 x 10¹⁰ pfu/ml with 99% recombinants;
Adult female: 1.4 x 10⁹ pfu/ml with 97% recombinants;
48 hour L3 larvae: 4.88 x 10⁹ pfu/ml with 97% recombinants;
6 day L4 larvae: 1.05 x 10⁹ pfu/ml with 98% recombinants.

These libraries were screened with pooled immune dog sera in a manner similar to that described in Example 4. The 48 hour L3 library was screened with immune dog serum prepared as described in Example 1 due to its almost exclusive reactivity with the 39 kD protein. Antibodies specific for a highly immunoreactive protein termed the "ladder protein" had been adsorbed from this serum by affinity chromatography as described in Example 4.

Development of immunoblots containing adult female worm, and L3 or L4 larval protein lysates with the adsorbed sera showed reactivity to the previously recognized 39 kD protein and one other >100 kD protein only in the larval lysates. Immunoscreening of duplicate plaque lifts of the 48 hour L3 library with this sera identified 4 positive clones named p39-1 through p39-4. p39-4 has been renamed p4 and is described in greater detail above. p39-1, p39-2 and p39-3 each represent p39 nucleic acid sequences that encode P39 proteins as defined herein.

An antibody select technique (Hall et al., 1984, *Nature* 311, 379-382) was used to obtain clone-specific antibodies for p39-1 and p39-3. Using the protocol described for library immunoscreening, two 90 mm agar plates, each containing 1.5 x 10⁴ purified phage were overlaid with nitrocellulose filters, previously soaked in 10 mM IPTG. The plaque lifts were incubated overnight in immune dog sera prepared as described in Example 1 diluted 1:200 in TBS-1% gelatin, and washed 4X in TBST (20 mM Tris-HCl, pH 7.5, 150 mM NaCl, 0.05% Tween-20) and 2X in TBS (TBST without the Tween-20). Like filters were treated with 2.0 ml glycine buffer (0.1 M glycine-HCl, pH 2.6, 0.15 M NaCl) for 15 minutes on ice to elute clone-specific antibodies. The acidic eluate was immediately neutralized by the dropwise addition of 1 M Tris, pH 8.0. The clone-specific antibodies were concentrated in an Amicon centricon 30 microconcentrator and used to develop immunoblots of *D. immitis* adult female, and L3 and L4 larval proteins. Clone-specific antibodies eluted from clones p39-1 and p39-3 detected a single 39 kD. larval specific protein that migrated at the same position as the larval 39 kD native protein selectively bound by immune dog serum. Antibodies selected from a non-recombinant phage showed no reactivity to the *D. immitis* proteins. These data indicate that these p39 recombinant clones encode the 39 kD protein selectively detected by immune dog sera. The clones identified as immunoreactive with the immune serum provide a source of DNA encoding desired proteins.

Double stranded plasmid DNA of clones p39-1, p39-2 and p39-3 was prepared as described in Example 4. Plasmid DNAs containing p39-1 and p39-2 were digested with *Eco*RI and *Xho*I restriction endonucleases to release two *D. immitis* DNA fragments of about 1000 and about 200 nucleotides, the entire *D. immitis* fragments each being about 1200 nucleotides in size. Digestion of plasmid DNAs containing p39-3 with *Eco*RI and *Xho*I restriction endonucleases released two *D. immitis* DNA fragments of about 1000 and about 60 nucleotides, the entire *D. immitis* fragment being about 1060 nucleotides in size.

Double stranded p39-1, p39-2 and p39-3 nucleic acid sequences were sequenced as described in Example 4. p39-1 and p39-2 include the following nucleic acid sequence (deduced start and stop codons underlined): p39-3 is similar to p39-1 and p39-2 except that p39-3 lacks the first 125 nucleotides of p39-1 and p39-2 and has an additional "A" after nucleotide 203 (referring to the numbering of the p39-1 and p39-2 sequences). All three p39 nucleic acid sequences appear to encode a protein of about 300 amino acids, having a start codon spanning about nucleotides 216 through 218 and a stop codon spanning about nucleotides 1116 through 1118 (referring to the same sequences). The deduced amino acid sequence of the P39 protein is reported above.

### Comparative Example 9

This Example demonstrates the ability of a *D. immitis* p39 nucleic acid sequence to encode a protein that selectively binds to immune serum.

Proteins encoded by DNA inserted in frame into the *Eco*RI site of pBluescript expression vector, as described in Example 8, have an additional 4 kD of beta galactosidase peptide attached to the N-terminus upon induction with IPTG. This peptide is encoded by the vector and allows for purification of the resulting fusion protein by affinity chromatography. *E. coli* cultures of each p39 clone were induced with IPTG to determine the size and quantity of fusion protein produced by each clone. Samples were collected prior to induction, and at 1 hour, 2 hours, 3 hours and 4 hours after induction. Clones p39-1 and p39-3 encoded fusion proteins of about 40 to about 43 kD, although p39-3 led to the production of more protein per cell weight than did p39-1 as evidenced by Coomassie blue straining of proteins separated by SDS-PAGE.

To verify the P39 fusion protein detected by SDS-PAGE reacted with the immune dog sera used to originally isolate the clones, a western blot containing either the p39-1 or the p39-3 *E. coli* lysates was developed with immune serum produced as in Example 1. A protein of about 40 to about 43 kD was detected in lysates of both clones but not in a nonrecombinant pBluescript lysate.

### Comparative Example 10

This Example also demonstrates the ability of a *D. immitis* p39 nucleic acid sequence to encode a protein that selectively binds to immune serum. Furthermore, monospecific antibodies that are selected by their ability to bind to the recombinant P39 protein are also capable of binding to 39 kD L3 and L4 antigens. Rabbits, cats and dogs have been immunized with this recombinant P39 protein.

Recombinant molecule pHis-p39₉₀₀, containing *D. immitis* p39-1 nucleotides from about 216 through about 1118 (referring to numbering in p39-1 and p39-2 nucleic acid sequences presented in Example 8) were operatively linked to *trc* transcription control sequences and to a fusion sequence encoding a poly-histidine segment comprising 6 histidines was produced in a manner similar to that described for the production of recombinant molecule p22U₆₀₈ in Example 7. Briefly, the specified p39 nucleic acid sequence was PCR amplified from a clone containing *D. immitis* p39-1 and the product subcloned into expression vector pTrcHisB. The resulting recombinant molecule, pHis-p39₉₀₀, was transformed into *E. coli* to form recombinant cell *E. coli*:pHis-p39₉₀₀; for details, see Example 11. The recombinant cell was cultured and induced as described in Example 7. Protein production was monitored by SDS PAGE of recombinant cell lysates, followed by Coomassie blue staining, using standard techniques. Recombinant cell *E. coli*:pHis-p39₉₀₀ produced a protein, denoted herein as PHIS-P39₉₀₀, that migrated with an apparent molecular weight of about 46 kD. Such a protein was not produced by cells transformed with the pTrcHisB plasmid lacking a *D. immitis* DNA insert.

Immunoblot analysis of recombinant cell *E. coli*:pHis-p39₉₀₀ lysates indicates that the 46-kD protein was able to selectively bind to immune dog serum and, as such, was capable of binding to at least one component of a serum that is capable of inhibiting *D. immitis* larval development. Immune dog serum essentially did not bind to lysates of cells transformed with only the pTrcHisB plasmid.

Purified PHIS-P39₉₀₀ fusion protein was incubated with immune dog sera produced as in Example 1, the monospecific antibodies eluted from the protein and reacted with Western blots of *D. immitis* life stage specific antigens. The clone-specific antibodies recognized a 39 kD larval-specific antigen in 0 hr L3, 48 hour L3, and 6 day L4 antigen preparations but did not detect this protein in adult male, female or microfilaria preparations. These data verify the 39 cDNA encodes a larval specific 39 kD protein recognized by immune dog sera.

A rabbit and 2 dogs have been immunized with the PHIS-P39₉₀₀ fusion protein using standard protocols, such as those disclosed above. Cats have also been immunized with the PHIS-P39₉₀₀ fusion protein.

### Comparative Example 11

This Example demonstrates the production of a P39 protein in eukaryotic cells as well as the production of a recombinant virus particle vaccine capable of expressing P39.

A PCR product was generated from p39-3 Unizap XR phage DNA (see Example 8) using primers 39CT (5' CGCGGATCCC GCAAATGAGA TCATG3') and 39COOH (5' GCCAACGGAT CCATTCAGTC AACATACC3'), which have *Bam*HI sites (underlined) incorporated into the primers. This approximately 900 bp PCR product was digested with *Bam*HI and subcloned into a *Bam*HI digested, CIP (calf intestine phosphatase)-treated pTrcHisB vector (available from Invitrogen). Proper 5' to 3' orientation of the p39 insert within the vector was verified. This clone is referred to as pRis-p39₉₀₀.

An *Xho*I restriction site was added to a pSP64 vector (available from Promega) by linearizing pSP64 with *Sma*I, ligating an *Xho*I linker to one end, and recircularizing the vector with T4 DNA ligase to form pSP64-Xho. The pSP64-Xho vector was digested with *Bam*HI, CIP-treated and ligated in the appropriate orientation to the *Bam*HI fragment containing the p39 sequence cleaved from pHis-p39₉₀₀. The resultant molecule is referred to as p76-80.B3.

In order to subclone p39 into baculovirus or sindbis expression vectors, p39-containing fragments were PCR amplified from p76-80.B3 DNA using an antisense primer 76-58.B (5' GTGGAATTGT GAGCGG3') homologous to pSP64 located downstream of the p39 sequence and an N-terminal primer designed from p39 sequence with modifications to enhance expression in the individual systems. Specifically, the N-terminal primer 105-47.A (5' GCGGGATCCT ATAAATATGA TAGATTTGAA GAAG3' having a *Bam*HI site (underlined)) was used to generate a p39-containing PCR product (referred to as BVp39) for subcloning into the baculovirus shuttle plasmid BlueBacIII (available from Invitrogen). An N-terminal primer 105-08.A (5' GCTCTAGACC ATGATAGATT TGAAGAAG3' having an *Xba*I site (underlined)) was used to produce a p39-containing PCR product (referred to as svp39) for subcloning into the Sindbis virus shuttle plasmid Toto2J1-minus.

In order to produce a baculovirus recombinant molecule capable of directing the production of P39₉₀₀, BVp39 was digested with *Bam*HI and ligated into the unique *Bam*HI site of BlueBacIII (available from Invitrogen) shuttle plasmid. The orientation of the insert was verified and the resultant recombinant molecule designated p105-72.5C. This recombinant molecule and linear Baculogold baculovirus DNA (available from Pharmingen, San Diego, CA) were cotransfected into *Spodoptera frugiperda* Sf9 host cells (donated by the Colorado Bioprocessing Center, Fort Collins, CO) to form *S. frugiperda*:p105-72.5C. The recombinant virus, denoted 105-92.1, was verified for proper insert orientation and cultivated for increased production of recombinant virus and to verify expression of P39₉₀₀ by Western blot. Immunoblot analysis indicates that P39₉₀₀ produced using baculovirus vectors is capable of being selectively bound by immune sera.

In order to produce a recombinant virus particle vaccine in which P39₉₀₀ expression is under the control of a Sindbis virus promoter, SVp39 was directionally ligated into the *Xba*I and *Xho*I restriction sites of Sindbis virus vector Toto2J1 following removal of the chloramphenicol acetyltransferse (CAT) gene from this location in the vector. The resulting recombinant molecule is denoted p105-42.1A. Note that Toto2J1 is a Sindbis virus expression vector that contains the SP6 RNA polymerase promoter and the entire Sindbis virus genome through to the *Nsi*I restriction site at nucleotide 11452 (i.e., each of the nonstructural polypeptide genes, the subgenomic promoter, and each of the structural polypeptide genes) ligated to an *Ssp*I (nucleotide position 7499)/*Sst*I restriction fragment from TRCAT62 which contains the subgenomic promoter, 14 nucleotides of the 5' untranslated sequence of the subgenomic mRNA, the CAT gene, 62 nucleotides of Sindbis virus 3' untranslated sequence, and the Sindbis virus poly-A sequence (see Xiong et al., 1989, *Science* 243, 1188-1191).

Recombinant molecule p105-42.1A was linearized by digestion with *Mlu*I, infectious recombinant Sindbis transcripts generated with SP6 RNA Polymerase and used to infect BHK (baby hamster kidney) host cells using techniques as described in Xiong et al., *ibid*, thereby forming BHK:p105-42.1A. The resulting recombinant virus (denoted 105-71.1) was cultivated for increased production and is analyzed for expression by Western blot. Immunoblot analysis indicates that P39₉₀₀ produced by this recombinant virus is capable of being selectively bound by immune sera. The infectious recombinant virus can be used as a live vaccine or in an expression system to produce P39.

### Example 12

This example describes the cloning and sequencing of p22L and p20.5 nucleic acid sequences.

Oligomeric DNA primers and probes were made by DNA Express, Department of Biochemistry, Colorado State University. Synthesis was done using an ABI model 392 DNA/RNA synthesizer using cyano-ethyl-phosphoramidite chemistry. Products were purified using low pressure reverse phase chromatography. Nucleic acid oligonucleotide probes were 3'-end labeled by tailing with (³²P)-deoxycytidine as described by Collins and Hunsaker, 1985, *Analyt. Biochem.* 151, 211-224. Terminal deoxynucleotidyl transferase (available from Promega Corp.) was used according to manufacturers instructions.

A 48-hour L3 cDNA library constructed from poly A+ selected RNA using the ZAP-cDNA® Synthesis Kit and the Uni-ZAP XR vector (both available from Stratagene) was produced as described in Example 4. The final library contained about 4.88 x 10⁶ plaque forming units (pfu)/µl with 96.4% recombinants. Library plating and plaque lifts were essentially done according to manufacturer's instructions (Stratagene) using XL1-Blue *Escherichia coli*; see also Sambrook et al., *ibid.*

Plaques lifted onto Nytran 0.45 µm 137 mm membranes (available from Schleicher and Schuell, Keene, New Hampshire) were hybridized under stringent conditions (see Sambrook et al., *ibid*.) with about 5 pmoles of labeled GRF6 probe (approximately 1.5 x 10⁶ cpm/ml). This probe was synthesized based on the amino acid sequence EACYDQ obtained from the 32 and 35 min tryptic peptides of the P20.5 and P22L proteins, respectively, described and shown in Example 3. The DNA sequence of GRF6 was 5' GAA GCI TGC TAT GAT CAA 3', where I is inosine which is capable of base pairing with all four bases. The wobble base selection for glutamic acid (E), cysteine (C), tyrosine (Y), aspartic acid (D) and glutamine (Q) was based on codon usage for two *D. immitis* proteins previously reported (GenBank accession numbers M29733 and M82811). Of about 102,000 plaques screened, approximately 252 (0.25%) hybridized to the probe. Sixteen plugs containing positive plaques were removed and 5 of these were plaque purified using standard techniques.

Double stranded plasmid DNAs of clones containing p22L or p20.5 nucleic acid sequences were prepared as described in Example 4 and submitted to restriction enzyme cleavage by *Eco*RI and *Xho*I restriction endonucleases. Insert sizes were estimated to be 334 bp (clone 1), 442 bp (clone 2), 603 bp (clone 3), 589 bp (clone 4) and 442 bp (clone 5), 32 bp of which were vector sequence.

DNA sequencing of the clones was accomplished as described in Example 4. Partial DNA sequence was determined for clones 1, 2, 3 and 5 and the full double strand sequence of the protein coding region was determined for clone 4. The nucleic acid sequences of the coding regions of p22L and p20.5 as well as the deduced amino acid sequences of P22L and P20.5 are presented above. (MacVector™ version 3.5 sequence analysis software (International Biotechnologies, Inc., New Haven, Connecticut) was used for amino acid translations, protein molecular weight and isoelectric point calculations, and hydrophilicity calculations).

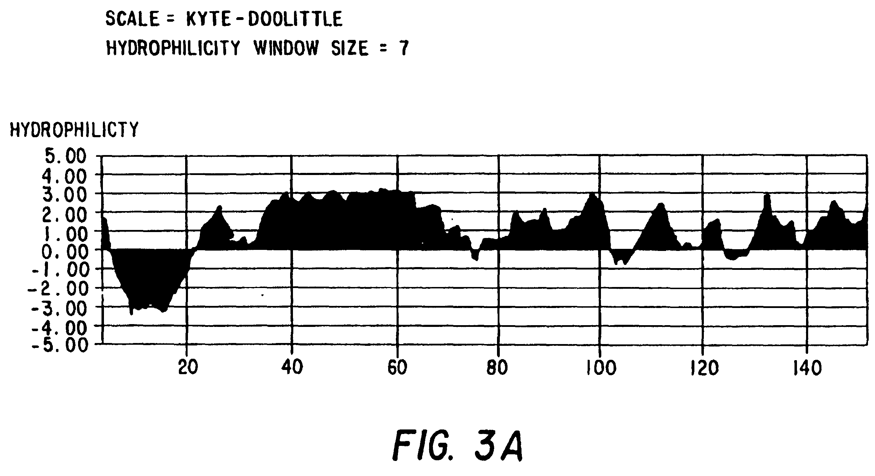

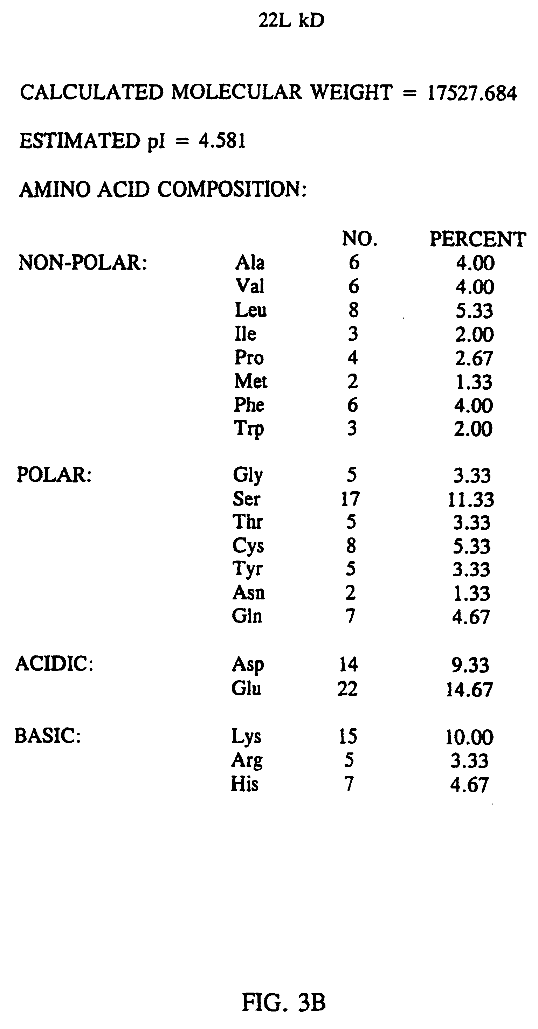

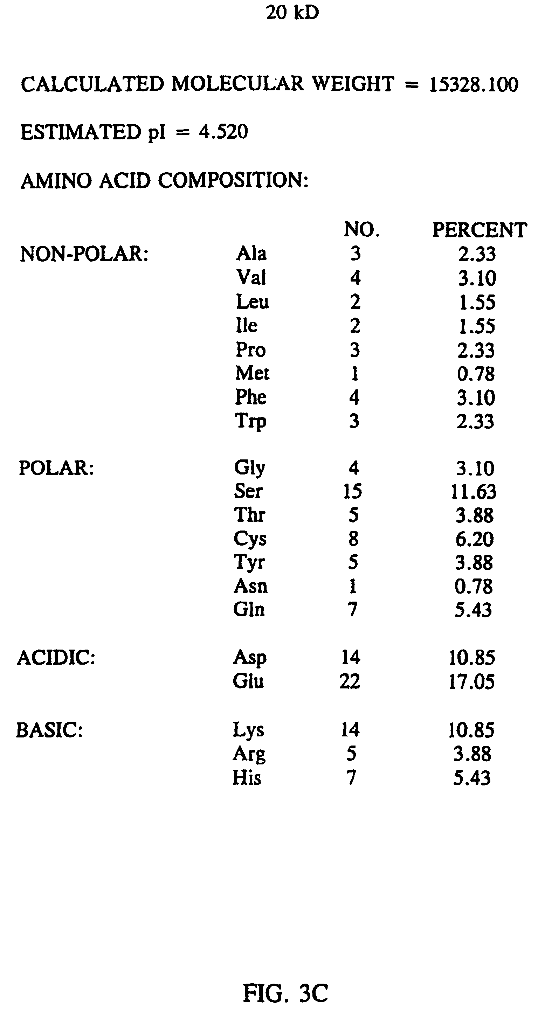

FIG. 3A shows a hydrophilicity plot of the amino acid sequences deduced for P22L and P20.5. Hydrophilicity was calculated based on the method of Kyte et al., 1982, *J. Mol. Biol.* 157, 105-132, with a window size of 7 amino acids. Molecular weights, isoelectric points (pI) and amino acid compositions of the entire P22L sequence (FIG. 3B, 22L kD) and the proposed P20.5 cleavage product beginning at the glutamic acid at position 22 (FIG. 3C, 20 kD) are shown.

The protein encoded by p22L is very hydrophilic with the exception of the N-terminal 21-amino acid hydrophobic leader that is apparently cleaved from P20.5. The calculated molecular weight of this 21 amino acid segment is 2.2 kD. This relationship between P22L and P20.5 explains both the similar chromatographic behavior and the similar immunological reactivity of the two molecules. The calculated molecular weight and pI of P22L are 17,527.7 and 4.58, respectively, while those for P20.5 are 15,328.1 and 4.52, respectively. These calculations differ from those obtained from routine Tris-glycine SDS-PAGE under reducing conditions. The calculated molecular weights are in the range calculated from Tris-tricine SDS-PAGE.

The calculated isoelectric points were 4.52 and 4.58 for the 15.3 and 17.5 kD (20 and 22L kD) proteins, respectively. Original estimates for the 20 and 22 kD proteins (Frank et al., *ibid*.) were that the 20 kD protein was acidic while the 22 kD protein was basic. This was prior to determining the presence of two proteins in the 22 kD region, namely P22U and P22L.

### Example 13

This example discloses Northern blot analysis of *D. immitis* L3 and L4 RNA using probes corresponding to a portion of p22L.

*D. immitis* 0-hour L3, 48-hour L3 and 6-day L4 larvae were harvested and total RNA purified therefrom as described in Example 4. Adult female poly A+ RNA was prepared similarly after the worms were ground to a fine powder in liquid nitrogen. RNA was electrophoresed through agarose-formaldehyde gels and transferred to Nytran membranes (available from Schleicher and Schuell) essentially as described by Sambrook et al., *ibid*. with minor modifications as described in Fourney et al., 1988, *Focus* 10, 5-6. Samples of about 10 micrograms of larval total RNAs and about 1.8 µg adult female poly A+ RNA were analyzed. An initial hybridization procedure resulted in excessive non-specific binding of probe which had to be stripped by standard techniques. RNA species immobilized on the membrane were then hybridized for about 52 hours using standard Northern blot conditions to about 5 x 10⁵ cpm/ml labeled GRF10. GRF10 was an anti-sense oligomeric DNA corresponding to nucleotides 5' CATAGTTCTT GGCTTAGCGC TTC 3' of the coding region of p22L (spanning about nucleotides 15 through 37 represented above). After washing and exposure, a strong signal was seen in the 48-hr L3 lane and a weaker signal was seen in the 6-day L4 lane each corresponding to RNA species of about 720 to about 730 bases. GRF10 essentially did not hybridize to 0-hr L3 or adult female RNA.

Although only 1.8 µg of RNA was loaded in the adult female lane, presumably this contained considerably more message than the 10 µg of larval total RNA per lane due to the poly A+ selection. The finding of a relative abundance of message in 48 hr L3, less in 6 day L4 and none in 0 hr L3, adults and, presumably, microfilariae, substantiates the pulse-chase metabolic labeling patterns described by Frank et al., *ibid.* and the apparent lack of these molecules while purifying the 22U kD protein from adults.

### Example 14

This Example demonstrates the ability of *D. immitis* p22L (also denoted *D. immitis* PLA2) to encode a protein that, when expressed in bacteria, selectively binds to immune serum. Furthermore, the recombinant protein can induce the production of antibodies in rabbits and dogs capable of recognizing the corresponding native and recombinant heartworm antigens. Cats have been immunized with the recombinant protein.

Recombinant molecule pET19b-PLA2₄₁₇, containing *D. immitis* p22L nucleotides from about base 58 through about base 474 operatively linked to bacteriophage T7*lac* transcription control sequences and to a fusion sequence encoding a poly-histidine segment comprising 10 histidines was produced in the following manner. An about 417-nucleotide DNA fragment containing nucleotides spanning from about 58 through about 474 of the nucleic acid sequence of the deduced coding region of p22L disclosed above, called PLA2₄₁₇, was PCR amplified from a clone containing *D. immitis* p22L using the primers 5' CGCGGATCCT TCCGCATCAG AATCACAAGA AG 3' (denoted 20 NH2; *Bam*HI site underlined) and 5' CGAAGGAATG GATCCTTATA AGTTATTAAT CG 3' (denoted 20 COOH'; *Bam*HI site underlined). The PCR product was digested with *Bam*HI restriction endonuclease, gel purified and subcloned into expression vector pET19b (available from Novagen Inc.) that had been cleaved with *Bam*HI. The resulting recombinant molecule pET19b-PLA2₄₁₇ was transformed into *E. coli* BL21(DE3)pLysS to form recombinant cell *E. coli*:pET19b-PLA2₄₁₇. *E. coli* BL21(DE3)pLysS includes a bacteriophage T7 RNA polymerase gene under the control of *lac* transcription control sequences.

Recombinant cell *E. coli*:pET19b-PLA2₄₁₇ was cultured and expression induced as described in Example 5. Protein production was monitored by SDS PAGE of recombinant cell lysates, followed by Coomassie blue staining, using standard techniques. Recombinant cell *E. coli*:pET19b-PLA2₄₁₇ produced a protein, denoted PHIS-PLA2₄₁₇, that migrated with an apparent molecular weight of about 26 kD. Such a protein was not produced by cells transformed with the pET-19b plasmid lacking a *D. immitis* DNA insert.

Immunoblot analysis of recombinant cell *E. coli*:pET19b-PLA2₄₁₇ lysates indicated that the 26 kD protein was able to selectively bind to immune dog serum and, as such, was capable of binding to at least one component of a serum that is capable of inhibiting *D. immitis* larval development.

The *E. coli*:pET19b-PLA2₄₁₇ histidine fusion peptide was separated from soluble *E. coli* proteins by nickel chelation chromatography and an imidazole gradient. Immunoblot analysis of the total *E. coli*:pET19b-PLA2₄₁₇ lysate, column eluate and column void volume indicated that the PHIS-PLA2₄₁₇ 26 kD protein can be isolated on the nickel column and was able to selectively bind to immune dog serum, and as such, was capable of binding to at least one component of a serum that is capable of inhibiting D. immitis larval development. The column eluate was not detected by preimmune sera from the same immune dog.

A rabbit was immunized twice with PHIS-PLA2₄₁₇ that was purified by chelation chromatography followed by C4 reverse phase chromatography. Antisera collected from this rabbit, denoted anti-PHIS-PLA2₄₁₇ antisera, was used to characterize the protein in the larvae.

Immunoblot analysis was performed on 1500 microfilaria, 150 each of 7 day mosquito derived L2 from malpighian tubules, 10 day mosquito derived L3 from heads, 15 day mosquito derived L3 from heads, 0 hr *in vitro* cultured L3 harvested at 25°C, 0 hr *in vitro* cultured L3 harvested at 37°C, 48 hr *in vitro* cultured L3, 6 day *in vitro* cultured L4, 10 day *in vitro* cultured L4, 13 day *in vitro* cultured L4, 3 µg adult male antigen and 3 µg adult female antigen. Immunoreactivity to *D. immitis* PLA2 (as detected using anti-PHIS-PLA2₄₁₇ antisera) was found in all L3 and L4 samples examined, but was not detected in microfilaria, L2 or adult males or females. Processing from the 22 kD form to the 20.5 kD form was also observed. The 22 kD form appeared in the L3 as early as 10 days in the mosquito, and remained as such until harvested for *in vitro* culture at day 15 in the mosquito. The 20 kD form was found as early as 48 hours after removal from the mosquito. Upon subsequent *in vitro* incubation, the 22 kD form gradually diminished until it was barely evident by 13 days in culture, while the 20.5 kD form was still clearly evident.

Immunoblot analysis, using anti-PHIS-PLA2₄₁₇ antisera, was performed on both P22L and P20.5 purified from *D. immitis* larval excretory-secretory products demonstrating both were immunologically recognized.

Two dogs were immunized three times with PHIS-PLA2₄₁₇ that was purified by chelation chromatography. Antisera collected from the dogs recognized both the P22L and P20.5 in 48 hr L3 *D. immitis.*

Fifteen cats were immunized twice with PHIS-PLA2₄₁₇ that was purified by chelation chromatography. These cats were challenged with 40 L3 each and are under evaluation for protection.

### Example 15

This example demonstrates the production of a P22L protein (also referred to as PLA2) in eukaryotic cells as well as the production of a recombinant virus particle vaccine capable of expressing P22L, the P22L being capable of selectively binding to immune serum.

A PCR product of about 475 nucleotides containing p22L sequences from nucleotide about 1 to about 475 (based on the p22L coding sequence disclosed above) was generated from a p22L-containing clone of Example 12 using primers 76-40.B (5' GCGGGATCCA ACATGAACAA ACTTTTCATA GTTC3') and 20COOH (5' CGAAGGAATG GATCCTTATA AGTTATTAAT CG3'), each of which have BamHI sites (underlined) incorporated into the primers. The *Bam*HI-digested p22L₄₇₅ PCR product was subcloned into a *Bam*HI-digested, CIP treated pSP64 vector (available from Promega) to form the vector p76-52.H3. Proper insert orientation was verified.

For subcloning into the baculovirus shuttle plasmid, BlueBacIII (available from Invitrogen), the p76-52.H3 plasmid DNA was digested with *Bam*HI and the p22L₄₇₅ insert DNA was subcloned into the unique *Bam*HI site of BlueBacIII. The resulting recombinant molecule is denoted p76-79-A6. Insert orientation was verified and p76-79-A6 plasmid DNA was cotransfected into Sf9 host cells (donated by the Colorado Bioprocessing Center, Fort Collins, CO) with linear Baculogold baculovirus DNA (available from Pharmingen) and insectin cationic liposomes (available from Invitrogen) to form *S. frugiperda*:p76-79-A6. The resulting recombinant p22L₄₇₅/baculovirus is denoted 89-11. Western blots using rabbit anti-PHIS-PLA2₄₁₇ antisera produced as described in Example 14 demonstrated that insect cells transfected with recombinant baculovirus 89-11 expressed and processed the protein encoded by p22L₄₇₅, namely P22L₄₇₅, also referred to as PLA2₄₇₅. Both the 22 and 20.5 kD forms of the protein were identified in whole cell lysates, but only the 20.5 kD form was secreted into the cell culture medium. This result indicated that the secretory signal of *D. immitis* P22L is recognized by insect cells and that the molecule is processed naturally in this system.

For subcloning into the Sindbis virus shuttle plasmid, Toto2J1 (described in Example 11), p76-52.H3 DNA was digested with *Bam*HI to release the p22L₄₇₅ insert DNA which was subcloned into the unique *Bam*HI site of pSP64-XhoI vector, produced as described in Example 11. Proper 5' to 3' orientation of the insert within the vector was verified. The recombinant molecule is referred to as p76-79.C2. The p76-79.C2 DNA was digested with *Xba*I and *Xho*I to release the p22L₄₇₅ insert which was directionally subcloned into the *Xba*I-*Xho*I sites of the Sindbis virus shuttle plasmid, Toto2J1 as described in Example 11. The resulting recombinant molecule is referred to as p88-36.1B.

Recombinant molecule p88-36.1B was linearized at a unique *Mlu*I site. Infectious recombinant Sindbis transcripts generated with SP6 RNA Polymerase and used to infect BHK (baby hamster kidney) host cells as described in Example 11 to produce BHK:p88-36.1B. The recombinant virus particle (termed 48-87) was cultivated for increased production. Western blot analysis of infected cell lysates using rabbit anti-PHIS-PLA2₄₁₇ antisera produced as described in Example 14 shows that mammalian cells transfected with recombinant Sindbis virus particle 48-87 expressed, but apparently does not process, the protein encoded by p22L₄₇₅, namely P22L₄₇₅, also referred to as PLA2₄₇₅.

### Example 16

This Example shows that P22U, but apparently not P22L or P20.5, is expressed in adult heartworms.

Twenty-eight adult female *D. immitis* that had been stored at -70°C were washed 3 times with PBS, comminuted and homogenized with a glass/teflon homogenizer in 40 mM NaCl, 7.5 mM potassium phosphate pH 6.0, 1 mM EDTA, 1 mM PMSF, 2 mM DTT, 80 µg/ml leupeptin, 80 µg/mi pepstatin and 1 mg/ml TAME. The homogenate was sonicated for a total of 1 min using a 418 probe attached to a W-380 sonicator (available from Heat System-Ultrasonics, Inc., Farmington, New York), centrifuged at 5,000 g for 10 min and the supernatant was collected.

Twenty four adult male *D. immitis* that had been stored at -70°C were washed 3 times with TBS (50 mM Tris, 150 mM NaCl pH 8.0), frozen in liquid nitrogen and ground to a fine powder with a mortar and pestle. This powder was homogenized in 40 mm NaCl, 20 mm Tris pH 7.2, 1 mM EDTA, 1 mM PMSF, 5 µg/ml leupeptin, 5 µg/ml pepstatin and 1 mg/ml TAME. The homogenate was centrifuged at 10,000 g for 20 min and the supernatant was collected.

The resultant supernatant materials of both male and female worms were further concentrated and the buffer was exchanged to 20 mM Tris, 1 mM EDTA ph 7.2 using Centriprep-10 and Centricon-10 (available from Amicon). All steps described were conducted at 4°C or on ice.

Similar purification procedures as described for larval ES to prepare proteins for trypsin digestion (e.g., cation exchange followed by reverse phase chromatography as described in Example 3) were conducted on both adult male and female somatic soluble extracts to determine if P22U, P22L or P20.5 could be found in adult heartworms.

A protein consistent with the P22U protein was found following C₄ reverse phase chromatography, but neither P22L or P20.5 was seen in the eluates. The eluted protein was subjected to trypsin digestion and the fragments separated as described in Example 3. Tryptic fragment maps of P22U from larval ES, adult female and adult male sources were virtually identical as were the N-terminal sequences of selected tryptic fragments eluting at the same position. It appears, therefore, that P22U is also found in adult *D. immitis*, while there is no clear evidence to suggest the presence of either P22L or P20.5 in adult somatic soluble preparations.

### Example 17

Recombinant or native larval peptides are used to immunize dogs for the purpose of obtaining specifically reactive blood components. Recombinant antigens are administered to dogs with or without adjuvant by the subcutaneous, intramuscular, intradermal or intravenous routes. Following single or multiple immunization, blood is collected from dogs by routine venipuncture. Serum is collected from coagulated blood and used directly or stored frozen prior to use. Leukocytes are collected from anticoagulant-treated blood by density gradient centrifugation and used directly or stored by freezing at 1°C/minute with storage in liquid nitrogen.

### SEQUENCE LISTING

The following Sequence Listing is submitted pursuant to 37 CFR §1.821. A copy in computer readable form is also submitted herewith. The paper and computer readable forms of this Sequence Listing are the same.
## Claims

1. An isolated parasitic helminth nucleic acid molecule comprising a nucleic acid sequence capable of hybridizing, under stringent conditions, to at least one *Dirofilaria immitis* (*D. immitis*) nucleic acid molecule selected from the group consisting of *D. immitis* nucleic acid sequence and *D. immitis* nucleic acid sequence

2. The isolated nucleic acid sequence of Claim 1, wherein said isolated nucleic acid sequence encodes a protein capable of selectively binding to at least one component of immune serum, said immune serum being capable of inhibiting helminth development.

3. The isolated nucleic acid sequence of Claim 1, wherein said parasitic helminth is selected from the group consisitng of *Dirofilaria, Onchocera, Brugia, Wuchereria, Loa, Acanthocheilonema, Dipetalonema, Setaria, Parafilaria* and *Stephanofilaria* filarial nematodes.

4. The isolated nucleic acid sequence of Claim 1, wherein said *D. immitis* nucleic acid molecule comprises a nucleic acid sequence selected from the group consisting of *D. immitis* nucleic acid sequence and and complements thereof.

5. A recombinant molecule comprising at least one isolated nucleic acid sequence set forth in any one of Claims 1-4 operatively linked to at least one transcription control sequence.

6. A recombinant cell comprising a cell transformed with at least one isolated nucleic acid sequence set forth in any one of Claims 1-4 in a manner such that said recombinant cell is capable of expressing said isolated nucleic acid sequence.

7. An isolated parasitic helminth protein capable of selectively binding to at least one component of immune serum that is capable of inhibiting helminth development, said protein being encoded by a parasitic helminth nucleic acid sequence capable of hybridizing, under stringent conditions, to a nucleic acid sequence complementary to a nucleic acid sequence selected from the group consisting of and

8. The protein of Claim 7, wherein said protein comprises an amino acid sequence selected from the group consisting of and

9. An isolated antibody capable of selectively binding to a parasitic helminth protein, said antibody being produced by a method comprising administering to an animal an effective amount of an isolated protein to produce said antibody, said protein being capable of selectively binding to at least one component of immune serum that is capable of inhibiting helminth development, and said protein being encoded by a parasitic helminth nucleic acid sequence capable of hybridizing, under stringent conditions, to a nucleic acid sequence complementary to a nucleic acid sequence selected from the group consisting of and

10. A therapeutic composition capable of protecting an animal from parasitic helminth infection when administered to said animal in an effective manner, said composition comprising an isolated parasitic helminth nucleic acid molecule comprising a nucleic acid sequence capable of hybridizing, under stringent conditions, to at least one *Dirofilaria immitis (D. immitis)* nucleic acid molecule selected from the group consisting of *D.immitis* nucleic acid sequence and *D.immitis* nucleic acid sequence

## Patentansprüche

1. Isoliertes Nukleinsäuremolekül eines parasitischen Wurms mit einer Nukleinsäuresequenz, die in der Lage ist, unter stringenten Bedingungen mit wenigstens einem *Dirofilaria immitis (D. immitis)*-Nukleinsäuremolekül zu hybridisieren, welches aus der Gruppe ausgewählt ist, bestehend aus der *D.immitis*-Nukleinsäuresequenz und der *D.immitis*-Nukleinsäuresequenz

2. Die isolierte Nukleinsäuresequenz nach Anspruch 1, wobei die isolierte Nukleinsäuresequenz ein Protein kodiert, welches in der Lage ist, selektiv an wenigstens einen Bestandteil eines Immunserums zu binden, wobei das Immunserum in der Lage ist, die Wurmentwicklung zu hemmen.

3. Die isolierte Nukleinsäuresequenz nach Anspruch 1, wobei der parasitische Wurm aus der Gruppe ausgewählt ist, bestehend aus den Filariennematoden *Dirofilaria, Onchocera, Brugia, Wuchereria, Loa, Acanthocheilonema, Dipetalonema, Setaria, Parafilaria* und *Stephanofilaria*.

4. Die isolierte Nukleinsäuresequenz nach Anspruch 1, wobei das *D.immitis*-Nukleinsäuremolekül eine Nukleinsäuresequenz umfaßt, die aus der Gruppe ausgewählt ist, bestehend aus der *D.immitis*-Nukleinsäuresequenz und und Komplementären davon.

5. Rekombinantes Molekül, welches wenigstens eine in einem der Ansprüche 1 bis 4 angegebene isolierte Nukleinsäuresequenz funktionsmäßig verknüpft mit wenigstens einer Transkriptionskontrollsequenz umfaßt.

6. Rekombinante Zelle, welche eine Zelle umfaßt, die mit wenigstens einer in einem der Ansprüche 1 bis 4 angegebenen isolierten Nukleinsäure in solch einer Art und Weise transformiert ist, daß die rekombinante Zelle in der Lage ist, die isolierte Nukleinsäuresequenz zu exprimieren.

7. Isoliertes Protein eines parasitischen Wurms, welches in der Lage ist, selektiv an wenigstens einen Bestandteil eines Immunserums zu binden, das in der Lage ist, die Wurmentwicklung zu hemmen, wobei das Protein von einer Nukleinsäuresequenz eines parasitischen Wurms kodiert wird, welche in der Lage ist, unter stringenten Bedingungen mit einer Nukleinsäuresequenz zu hybridisieren, welche zu einer Nukleinsäuresequenz komplementär ist, die aus der Gruppe ausgewählt ist, bestehend aus und

8. Protein nach Anspruch 7, wobei das Protein eine Aminosäuresequenz umfaßt, die aus der Gruppe ausgewählt ist, bestehend aus und

9. Isolierter Antikörper, der in der Lage ist, selektiv an ein Protein eines parasitischen Wurrns zu binden, wobei der Antikörper nach einem Verfahren hergestellt wird, welches Verabreichung einer wirksamen Menge eines isolierten Proteins an ein Tier zur Herstellung des Antikörpers umfaßt, wobei das Protein in der Lage ist, selektiv an wenigstens einen Bestandteil eines Immunserums zu binden, das in der Lage ist, die Wurmentwicklung zu hemmen, und wobei das Protein von einer Nukleinsäuresequenz eines parasitischen Wurms kodiert wird, welche in der Lage ist, unter stringenten Bedingungen an eine Nukleinsäuresequenz zu hybridisieren, welche zu einer Nukleinsäuresequenz komplementär ist, die aus der Gruppe ausgewählt ist, bestehend aus und

10. Therapeutische Zusammensetzung, welche in der Lage ist, ein Tier gegen eine parasitische Wurminfektion zu schützen, wenn sie dem Tier in einer wirksamen Art und Weise verabreicht wird, wobei die Zusammensetzung ein isoliertes Nukleinsäuremolekül eines parasitischen Wurms umfaßt, welches eine Nukleinsäuresequenz einschließt, die in der Lage ist, unter stringenten Bedingungen an wenigsten ein *Dirofilaria immits (D..immitis)*-Nukleinsäuremolekül zu hybridisieren, das aus der Gruppe ausgewählt ist, bestehend aus der *D.immitis*-Nukleinsäuresequenz und *D.immitis*-Nukleinsäuresequenz

## Revendications

1. Molécule d'acide nucléique isolée d'helminthe parasite, comprenant une séquence d'acide nucléique apte à l'hybridation, dans des conditions drastiques, à au moins une molécule d'acide nucléique de *Dirofilaria immitis* (*D. immitis*) choisie dans le groupe consistant en la séquence d'acide nucléique de *D. immitis* et la séquence d'acide nucléique de *D. immitis*

2. Séquence d'acide nucléique isolée suivant la revendication 1, ladite séquence d'acide nucléique isolée codant pour une protéine capable de se lier sélectivement à au moins un constituant d'un sérum immun, ledit sérum immun étant capable d'inhiber le développement d'helminthes.

3. Séquence d'acide nucléique isolée suivant la revendication 1, dans laquelle ledit helminthe parasite est choisi dans le groupe consistant en les nématodes filariens *Dirofilaria, Onchocera, Brugia, Wuchereria, Loa, Acanthocheilonema, Dipetalonema, Setaria, Para filaria* et *Stephanofilaria.*

4. Séquence d'acide nucléique isolée suivant la revendication 1, dans laquelle ladite molécule d'acide nucléique de *D. immitis* comprend une séquence d'acide nucléique choisie dans le groupe consistant en la séquence d'acide nucléique de *D. immitis* et et leurs compléments.

5. Molécule recombinante comprenant au moins une séquence d'acide nucléique isolée décrite dans l'une quelconque des revendications 1 à 4 liée de manière fonctionnelle à au moins une séquence de régulation de transcription.

6. Cellule recombinante comprenant une cellule transformée avec au moins une séquence d'acide nucléique isolée décrite dans l'une quelconque des revendications 1 à 4 de telle sorte que ladite cellule recombinante soit apte à l'expression de ladite séquence d'acide nucléique isolée.

7. Protéine isolée d'helminthe parasite capable de se lier sélectivement à au moins un constituant d'un sérum immun qui est capable d'inhiber le développement d'helminthes, ladite protéine étant codée par une séquence d'acide nucléique d'helminthe parasite apte à l'hybridation, dans des conditions drastiques, avec une séquence d'acide nucléique complémentaire d'une séquence d'acide nucléique choisie dans le groupe consistant et

8. Protéine suivant la revendication 7, ladite protéine comprenant une séquence d'amino-acides choisie dans le groupe consistant en et

9. Anticorps isolé capable de se lier sélectivement à une protéine d'helminthe parasite, ledit anticorps étant produit par un procédé comprenant l'administration à un animal d'une quantité efficace d'une protéine isolée pour produire ledit anticorps, ladite protéine étant capable de se lier sélectivement à au moins un constituant d'un sérum immun qui est capable d'inhiber le développement d'helminthes, et ladite protéine étant codée par une séquence d'acide nucléique d'helminthe parasite apte à l'hybridation, dans des conditions drastiques, avec une séquence d'acide nucléique complémentaire d'une séquence d'acide nucléique choisie dans le groupe consistant en et

10. Composition thérapeutique capable de protéger un animal contre une infection par des helminthes parasites lors de son administration audit animal d'une manière efficace, ladite composition comprenant une molécule d'acide nucléique isolée d'helminthe parasite comprenant une séquence d'acide nucléique apte à l'hybridation, dans des conditions drastiques, avec au moins une molécule d'acide nucléique de *Dirofilaria immitis (D. immitis)* choisie dans le groupe consistant en la séquence d'acide nucléique de *D. immitis* et la séquence d'acide nucléique de *D. immitis*


(1) GENERAL INFORMATION:
   (i) APPLICANT:
      Tripp, Cynthia A.
      Frank, Glenn R.
      Grieve, Robert B.
   (ii) TITLE OF INVENTION: NOVEL PARASITIC HELMINTH PROTEINS
   (iii) NUMBER OF SEQUENCES: 4
   (iv) CORRESPONDENCE ADDRESS:
      (A) ADDRESSEE: Sheridan Rose & McIntosh
      (B) STREET: 1700 Lincoln Street, #3500
      (C) CITY: Denver
      (D) STATE: CO
      (E) COUNTRY: U.S.A.
      (F) ZIP: 80203
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US not yet assigned
      (B) FILING DATE: 19-AUG-1993
      (C) CLASSIFICATION:
   (vii) PRIOR APPLICATION DATA:
      (A) APPLICATION NUMBER: US 08/003,257
      (B) FILING DATE: 12-JAN-1993
   (vii) PRIOR APPLICATION DATA:
      (A) APPLICATION NUMBER: US 08/003,389
      (B) FILING DATE: 12-JAN-1993
   (vii) PRIOR APPLICATION DATA:
      (A) APPLICATION NUMBER: US 07/654,226
      (B) FILING DATE: 12-FEB-1991
   (viii) ATTORNEY/AGENT INFORMATION:
      (A) NAME: Kovarik, Joseph E.
      (B) REGISTRATION NUMBER: 33,005
      (C) REFERENCE/DOCKET NUMBER: 2618-13
   (ix) TELECOMMUNICATION INFORMATION:
      (A) TELEPHONE: (303) 863-9700
      (B) TELEFAX: (303) 863-0223
      (C) TELEX: 467377
(2) INFORMATION FOR SEQ ID NO:1:
   (i) SEQUENCE CHARACTERISTICS:
      (A) LENGTH: 913 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: *Dirofilaria immitis*
      (D) DEVELOPMENTAL STAGE: Larva
   (vii) IMMEDIATE SOURCE:
      (A) LIBRARY: L3 and/or L4 larval *D. immitis* cDNA expression library
      (B) CLONE: p4
   (ix) FEATURE:
      (A) NAME/KEY: CDS
      (B) LOCATION: 3..911
   (xi) SEQUENCE DESCRIPTION: SEQ ID NO:1:
   GC GAG TTA AAT AGT CGA ATT TCC GGA GTA CAC CGT AAT ACT GCA GGT        47
      Glu Leu Asn Ser Arg Ile Ser Gly Val His Arg Asn Thr Ala Gly
       1               5                  10                  15
   GCT TTA CAA CGA TTT GCT CTA AAT GGT CAA AAT ACT CTT AAC GAA GGA       95
   Ala Leu Gln Arg Phe Ala Leu Asn Gly Gln Asn Thr Leu Asn Glu Gly
                   20                  25                  30
   TCA AGT TAT GAG CCA AAC GGA CTA TTT GTA TTT TCA GCA ATA AAC GGT      143
   Ser Ser Tyr Glu Pro Asn Gly Leu Phe Val Phe Ser Ala Ile Asn Gly
               35                  40                  45
   AGC CAT ACT GAT AGC TTA TCT CAG TAT GGT GAA GGA ATA AAT GAA AAT      191
   Ser His Thr Asp Ser Leu Ser Gln Tyr Gly Glu Gly Ile Asn Glu Asn
           50                  55                  60
   TAT CAT TCT GGA ACT AAT TAT TAT GAT GAA GTA GAA TTA AGA GAT AAA      239
   Tyr His Ser Gly Thr Asn Tyr Tyr Asp Glu Val Glu Leu Arg Asp Lys
       65                  70                  75
   ACA AAT CAG ACA TCG TAC ATT AAT GGA AAT GAT AAT GGA ATC AAT GGA      287
   Thr Asn Gln Thr Ser Tyr Ile Asn Gly Asn Asp Asn Gly Ile Asn Gly
   80                  85                  90                  95
   AAG GAT GAT GAA GAT CTG GAT GAA TGC TCT GAT CAA GAA TTC CGA TGT      335
   Lys Asp Asp Glu Asp Leu Asp Glu Cys Ser Asp Gln Glu Phe Arg Cys
                   100                 105                 110
   CCA TAT CTA GCT AAA ACA CTT TGT GTT CAT TAT TTG AAA ATA TGC GAT      383
   Pro Tyr Leu Ala Lys Thr Leu Cys Val His Tyr Leu Lys Ile Cys Asp
               115                 120                 125
   GGT ATT GAT GAT TGT GGT GAT GGA AGT GAT GAA ATG AAC TGT GCT GAT      431
   Gly Ile Asp Asp Cys Gly Asp Gly Ser Asp Glu Met Asn Cys Ala Asp
           130                 135                 140
   GAT GAA GTG ATA ACA TCA ATA AAT GGT AAC GAA TCA ATC AAT ATC AGA      479
   Asp Glu Val Ile Thr Ser Ile Asn Gly Asn Glu Ser Ile Asn Ile Arg
       145                 150                 155
   TGT GAT CCG GAT CAA TTT CGA TGT GAA AAT GGA AAA TGT ATC GCA CAA      527
   Cys Asp Pro Asp Gln Phe Arg Cys Glu Asn Gly Lys Cys Ile Ala Gln
   160                 165                 170                 175
   ATT GAT CGA TGT AAT CGA AAA TAT GAT TGT GAT GAT GGT ACA GAT GAA      575
   Ile Asp Arg Cys Asn Arg Lys Tyr Asp Cys Asp Asp Gly Thr Asp Glu
                   180                 185                 190
   ACA ACT TGT GAA TAT TTC GTG CAA GCT TTG CAA CAA GCG AGA GGT GTA      623
   Thr Thr Cys Glu Tyr Phe Val Gln Ala Leu Gln Gln Ala Arg Gly Val
               195                 200                 205
   ACG GTG CAG GAT AAT GCA ATT CGA GAT GAC GAG ATA CCA AAT TAT ACT      671
   Thr Val Gln Asp Asn Ala Ile Arg Asp Asp Glu Ile Pro Asn Tyr Thr
           210                 215                 220
   GTA TCC ATG GAA CAG AAA TAC GAT CAA GTA AAG GAA GAT AAG GAG CGG      719
   Val Ser Met Glu Gln Lys Tyr Asp Gln Val Lys Glu Asp Lys Glu Arg
       225                 230                 235
   CGA ATG CAA GAG GAG GAG GAA CAG GAA AGG CTG AGA GAG TAC GAG GAA      767
   Arg Met Gln Glu Glu Glu Glu Gln Glu Arg Leu Arg Glu Tyr Glu Glu
   240                 245                 250                 255
   CAG ATA CAG GAA AAA TTG AGG CAG GAG GAA GAA AGA GAA CGG CAA GAA      815
   Gln Ile Gln Glu Lys Leu Arg Gln Glu Glu Glu Arg Glu Arg Gln Glu
                   260                 265                 270
   CAG GAA AGA AGA CAA AAG GAA CGA GAA AGA ATG GAA CAA GAA AGG ATA      863
   Gln Glu Arg Arg Gln Lys Glu Arg Glu Arg Met Glu Gln Glu Arg Ile
               275                 280                 285
   AGA CAA GAA TAT GAT GAA AAG GAA AGA CAA AGG CAA TAT GCT GAA CAG      911
   Arg Gln Glu Tyr Asp Glu Lys Glu Arg Gln Arg Gln Tyr Ala Glu Gln
           290                 295                 300
   GC                                                                    913
(2) INFORMATION FOR SEQ ID NO:2:
   (i) SEQUENCE CHARACTERISTICS:
      (A) LENGTH: 303 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Glu Leu Asn Ser Arg Ile Ser Gly Val His Arg Asn Thr Ala Gly Ala
   1               5                   10                  15
   Leu Gln Arg Phe Ala Leu Asn Gly Gln Asn Thr Leu Asn Glu Gly Ser
               20                  25                  30
   Ser Tyr Glu Pro Asn Gly Leu Phe Val Phe Ser Ala Ile Asn Gly Ser
           35                  40                  45
   His Thr Asp Ser Leu Ser Gln Tyr Gly Glu Gly Ile Asn Glu Asn Tyr
       50                  55                  60
   His Ser Gly Thr Asn Tyr Tyr Asp Glu Val Glu Leu Arg Asp Lys Thr
   65                  70                  75                  80
   Asn Gln Thr Ser Tyr Ile Asn Gly Asn Asp Asn Gly Ile Asn Gly Lys
                   85                  90                  95
   Asp Asp Glu Asp Leu Asp Glu Cys Ser Asp Gln Glu Phe Arg Cys Pro
               100                 105                 110
   Tyr Leu Ala Lys Thr Leu Cys Val His Tyr Leu Lys Ile Cys Asp Gly
           115                 120                 125
   Ile Asp Asp Cys Gly Asp Gly Ser Asp Glu Met Asn Cys Ala Asp Asp
       130                 135                 140
   Glu Val Ile Thr Ser Ile Asn Gly Asn Glu Ser Ile Asn Ile Arg Cys
   145                 150                 155                 160
   Asp Pro Asp Gln Phe Arg Cys Glu Asn Gly Lys Cys Ile Ala Gln Ile
                   165                 170                 175
   Asp Arg Cys Asn Arg Lys Tyr Asp Cys Asp Asp Gly Thr Asp Glu Thr
               180                 185                 190
   Thr Cys Glu Tyr Phe Val Gln Ala Leu Gln Gln Ala Arg Gly Val Thr
           195                 200                 205
   Val Gln Asp Asn Ala Ile Arg Asp Asp Glu Ile Pro Asn Tyr Thr Val
       210                 215                 220
   Ser Met Glu Gln Lys Tyr Asp Gln Val Lys Glu Asp Lys Glu Arg Arg
   225                 230                 235                 240
   Met Gln Glu Glu Glu Glu Gln Glu Arg Leu Arg Glu Tyr Glu Glu Gln
                   245                 250                 255
   Ile Gln Glu Lys Leu Arg Gln Glu Glu Glu Arg Glu Arg Gln Glu Gln
               260                 265                 270
   Glu Arg Arg Gln Lys Glu Arg Glu Arg Met Glu Gln Glu Arg Ile Arg
           275                 280                 285
   Gln Glu Tyr Asp Glu Lys Glu Arg Gln Arg Gln Tyr Ala Glu Gln
       290                 295                 300
(2) INFORMATION FOR SEQ ID NO:3:
   (i) SEQUENCE CHARACTERISTICS:
      (A) LENGTH: 1016 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: *Dirofilaria immitis*
   (vii) IMMEDIATE SOURCE:
      (A) LIBRARY: adult female *D. immitis* cDNA expression library
      (B) CLONE: p22U
   (ix) FEATURE:
      (A) NAME/KEY: CDS
      (B) LOCATION: 3..629
   (ix) FEATURE:
      (A) NAME/KEY: 3'UTR
      (B) LOCATION: 630..1016
   (xi) SEQUENCE DESCRIPTION: SEQ ID NO:3:
   GT TTT GTT GTA CTA CTC GTT GTT GCA ATA TGG ATT GAA ATG AGC CAA       47
      Phe Val Val Leu Leu Val Val Ala Ile Trp Ile Glu Met Ser Gln
       1               5                  10                  15
   GGC CAA CAA ATG ATC AAA CAA TGT AAA TGT TCT GAT ATT GCA CCA TGT      95
   Gly Gln Gln Met Ile Lys Gln Cys Lys Cys Ser Asp Ile Ala Pro Cys
                    20                  25                  30
   CAA TTA ACT GCC GTT CAA TCA GTT TTA CCA TGT GCT GAT CAA TGC CAG     143
   Gln Leu Thr Ala Val Gln Ser Val Leu Pro Cys Ala Asp Gln Cys Gln
                35                  40                  45
   AAA TAT ATT ACT TCA ATT GGT GGT AAT TAT GAT CAA ATT AGT AAC TGT     191
   Lys Tyr Ile Thr Ser Ile Gly Gly Asn Tyr Asp Gln Ile Ser Asn Cys
            50                  55                  60
   TTT AAA CAG AAA CAA TCA ATT ATA AAT GAT GCT ATG AAA TGT GCT CAA     239
   Phe Lys Gln Lys Gln Ser Ile Ile Asn Asp Ala Met Lys Cys Ala Gln
        65                  70                  75
   GAT GCT TTC CCA AAT GCA TGC GCA CAA GGT GAA CCA AAA ATG GTA CCA     287
   Asp Ala Phe Pro Asn Ala Cys Ala Gln Gly Glu Pro Lys Met Val Pro
    80                  85                  90                  95
   AAA CGA TTC GGA AAA GGT CTT CAA TTA GCT GTA ATG ACT GAT ATC AAC     335
   Lys Arg Phe Gly Lys Gly Leu Gln Leu Ala Val Met Thr Asp Ile Asn
                   100                 105                 110
   AAA GAA TTA CAA CGA ATG GGA ATA GCA AAT CAA GTT ACT CAA CTA ATC     383
   Lys Glu Leu Gln Arg Met Gly Ile Ala Asn Gln Val Thr Gln Leu Ile
               115                 120                 125
   TCC CAA GGT CGA CGA TTC TTT AAA TGC TTC CAA TCG TGT ATG ATG AAA      431
   Ser Gln Gly Arg Arg Phe Phe Lys Cys Phe Gln Ser Cys Met Met Lys
           130                 135                 140
   AAA TTG GGC TCA TGT TCT CCA GAT TGT GGT CTT GAT TTA CCA TCT GAT      479
   Lys Leu Gly Ser Cys Ser Pro Asp Cys Gly Leu Asp Leu Pro Ser Asp
       145                 150                 155
   AAT GTT ATG GTT CAA ACA GTT AAA AAT TGC GCT CAA AAA AGT GGT ATT      527
   Asn Val Met Val Gln Thr Val Lys Asn Cys Ala Gln Lys Ser Gly Ile
   160                 165                 170                 175
   CAA ACT GCA TCG GTG CAA GAT CTT TGC TTT TGC GTC GAA CAA GCT GGT      575
   Gln Thr Ala Ser Val Gln Asp Leu Cys Phe Cys Val Glu Gln Ala Gly
                   180                 185                 190
   ATT CGG CAA CTT TCT GAT GTA TGT CCT CGT ATA CAA ATT TTC AAA ACG      623
   Ile Arg Gln Leu Ser Asp Val Cys Pro Arg Ile Gln Ile Phe Lys Thr
               195                 200                 205
   AAA TGAGTATTGA GAATATTGCA CTAGCAGCAA TCATTATTTT TCTCGAGAAT           676
   Lys
   TTTCGCTATC AATAAGTTGG AATATGATTA CAATAATATA TATATTAACT GCAAAAATCT    736
   TTCTTCTTCA AAATTATTTT TCATTTCGCT CTCATAATTG CATGATAATA GTCATAATGA    796
   AAAACAGGTT TTCTTTTTTT AAAATGATAA CTTCAAACAA ATAGGTATTT CTTGATATAT    856
   ATATGTATGT ATGTATGTGT GTGTGTGTGT GTGTGTGTAT GTGTGTGTTT GTGTATGTGT    916
   ATATGTATGT ATGTATGTAT GTATGTATGT ATGTGTAGGA GAAAAGCAAA CTAAACAGTA    976
   AATGAAAGAA AAAAATAAGT CAAATAAAAG TTTGATAATT                         1016
(2) INFORMATION FOR SEQ ID NO:4:
   (i) SEQUENCE CHARACTERISTICS:
      (A) LENGTH: 208 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Phe Val Val Leu Leu Val Val Ala Ile Trp Ile Glu Met Ser Gln Gly
    1               5                  10                  15
   Gln Gln Met Ile Lys Gln Cys Lys Cys Ser Asp Ile Ala Pro Cys Gln
               20                  25                  30
   Leu Thr Ala Val Gln Ser Val Leu Pro Cys Ala Asp Gln Cys Gln Lys
           35                  40                  45
   Tyr Ile Thr Ser Ile Gly Gly Asn Tyr Asp Gln Ile Ser Asn Cys Phe
       50                  55                  60
   Lys Gln Lys Gln Ser Ile Ile Asn Asp Ala Met Lys Cys Ala Gln Asp
   65                  70                  75                  80
   Ala Phe Pro Asn Ala Cys Ala Gln Gly Glu Pro Lys Met Val Pro Lys
                   85                  90                  95
   Arg Phe Gly Lys Gly Leu Gln Leu Ala Val Met Thr Asp Ile Asn Lys
               100                 105                 110
   Glu Leu Gln Arg Met Gly Ile Ala Asn Gln Val Thr Gln Leu Ile Ser
           115                 120                 125
   Gln Gly Arg Arg Phe Phe Lys Cys Phe Gln Ser Cys Met Met Lys Lys
       130                 135                 140
   Leu Gly Ser Cys Ser Pro Asp Cys Gly Leu Asp Leu Pro Ser Asp Asn
   145                 150                 155                 160
   Val Met Val Gln Thr Val Lys Asn Cys Ala Gln Lys Ser Gly Ile Gln
                   165                 170                 175
   Thr Ala Ser Val Gln Asp Leu Cys Phe Cys Val Glu Gln Ala Gly Ile
               180                 185                 190
   Arg Gln Leu Ser Asp Val Cys Pro Arg Ile Gln Ile Phe Lys Thr Lys
           195                 200                 205